# EUROPÄISCHE PATENTSCHRIFT

(11) **EP 2 014 251 B1**
(45) Veröffentlichungstag und Bekanntmachung des Hinweises auf die Patenterteilung: **30.05.2012**
(21) Anmeldenummer: 07112127.1
(22) Anmeldetag: 10.07.2007
(51) Int. Cl.: A61B 19/00, A61B 17/86

(54) **Befestigungsmittel zur Lagefixierung eines Körpers für medizinische Zwecke aus Polyphenylen und Befestigungsmittel zur Lagefixierung eines Körpers für medizinische Zwecke aus einer Siliziumnitrid-Keramik**
Attachment device for securing a body made of polyphenyls in position for medical purposes and attachment device for securing a silicon nitride ceramic body in position for medical purposes
Moyen de fixation destiné à la fixation en place d'un corps à des fins médicales en polyphényles et moyen de fixation destiné à la fixation en place d'un corps à des fins médicales en céramique-nitrite de silicium

(43) Veröffentlichungstag der Anmeldung: 14.01.2009
(73) Patentinhaber: BrainLAB AG, 85622 Feldkirchen (DE)
(72) Erfinder: Edlauer, Martin, 80809, München (DE); Radina, Christian, 85748, Garching (DE)
(74) Vertreter: Schwabe - Sandmair - Marx

(56) Entgegenhaltungen:
- EP-A- 1 026 513
- EP-A- 1 598 028
- WO-A-2006/127392
- US-A- 5 122 132
- US-A1- 2002 042 618

## Beschreibung

### Gebiet der Erfindung

Die vorliegende Erfindung bezieht sich auf ein Befestigungsmittel zur Lagefixierung eines Körpers, insbesondere eines Kopfes, für medizinische Zwecke, insbesondere bei Operationen, wobei das Befestigungsmittel wenigstens teilweise aus einem bestimmten Material hergestellt ist. Bei diesen Materialien handelt es sich zum einen um Polyphenylene und zum anderen um Siliziumnitrid-Keramiken.

### Stand der Technik

Aus dem Stand der Technik sind bereits Befestigungsmittel zur Lagefixierung eines Körpers, insbesondere eines Kopfes für medizinische Zwecke bekannt. Diese Befestigungsmittel werden verwendet, um insbesondere während Operationen eine feste Lagefixierung eines Körperteiles, insbesondere des Kopfes, zu gewährleisten. Dies ist insbesondere bei Operationen erforderlich, bei denen an die Exaktheit der Durchführung besonders hohe Anforderungen gestellt werden, z.B. bei Operationen im Gehirn, während denen z.B. ein Tumor entfernt werden soll. Selbst kleinste Abweichungen von der geplanten Schnittführung hätten bei solchen Operationen möglicherweise extrem negative Folgen für den Patienten, da ungewollt wichtige Abschnitte im Gehirn verletzt und ihrer Funktionsfähigkeit beraubt werden könnten. Um dieses Risiko zu minimieren, ist es wichtig, den Kopf eines Patienten während einer solchen cranialen Operation extrem fest zu fixieren.

Gemäß dem Stand der Technik werden für solche Zwecke Kopfklemmen mit bestimmten Befestigungsmitteln verwendet. Bei diesen Kopfklemmen handelt es sich um eine bügelförmige Vorrichtung, die an mindestens drei Punkten einen Vorsprung aufweist, an dem sich jeweils ein mit einer Spitze versehener Körper befindet, um den Kopf des Patienten an der Kopfklemme zu fixieren. Für gewöhnlich werden Drei-Punkt-Fixierungen oder Vier-Punkt-Fixierungen verwendet. Bei Drei-Punkt-Fixierungen befindet sich ein Fixierungspunkt auf der einen Kopfseite und die beiden anderen Fixierungspunke auf der anderen Kopfseite. Bei Vier-Punkt-Fixierungen befindet sich je ein Fixierungspunkt auf jeder Kopfseite, zwei weitere stützen bzw. fixieren den Kopf von unten. Zur Fixierung ist es nicht ausreichend, Befestigungsmittel zu verwenden, die lediglich von außen - d.h. ohne die Haut und den darunter liegenden Knochen zu durchtrennen bzw. einzuschneiden - wirken. Eine derartige Fixierung hat sich als nicht lagefest genug erwiesen. So sind z. B. stereotaktische Rahmen mit entsprechenden Befestigungsmitteln für Eingriffe der klassischen Neurochirurgie (Entfernen z. B. von Tumoren, Abszessen oder Gefäßmissbildungen) ungeeignet. Stattdessen werden in Kombination mit Kopfklemmen sog. Pins als Befestigungsmittel verwendet, das sind kleine stäbchenförmige Körper, die mit einer Spitze versehen sind, die an der Kopfklemme befestigt sind, und die in den Kopf, der fixiert werden soll, hineingesteckt werden. Dabei werden die Pins durch Drehen an einer Fixierschraube mittels einer translatorischen Bewegung durch die Kopfhaut in den Schädelknochen getrieben.

Gemäß dem Stand der Technik werden die konventionellen Pins, d.h. die Befestigungsmittel, die in die Kopfklemmen eingesetzt werden, aus einem metallischen Material hergestellt. Gängige Materialien sind z.B. Edelstahl und Titanlegierungen. Dies hängt damit zusammen, dass diese Materialien erwiesenermaßen eine verhältnismäßig gute mechanische Beanspruchbarkeit aufweisen.

EP-A-1 026 513 offenbart eine Halterung für einen Patientenkopf zur Verwendung bei Magnetresonanztomographie, deren Grundgerüst aus einem spritzgegossenem thermoplastischen Material, insbesondere Polyphenylsulfon gefertigt sein kann. Die Kopfhalterung kann ferner Pins 138 aufweisen, die in den Patientenschädel eingreifen.

EP-A-1 598 028 bezieht sich auf ein zahnmedizinisches Implantat, das aus Polyetheretherketon (PEEK), Polyphenylensulfon (PPSU) und/oder Polyethersulfon (PES), gegebenenfalls mit Faserverstärkung aus Aluminiumoxid, Siliziumoxid oder Zirkonoxid, hergestellt sein kann.

WO 2006/127392 A bezieht sich auf ein Implantat zur Fixierung der Wirbelsäule, das zur Verbindung und/oder mechanischen Immobilisierung benachbarter Wirbel verwendet werden kann.

Die mechanische Beanspruchbarkeit ist allerdings nicht das einzige Kriterium, das es bei der Materialauswahl für ein Befestigungsmittel zur Lagefixierung eines Körpers, insbesondere eines Kopfes, für medizinische Zwecke, insbesondere bei Operationen, zu beachten gilt. Insgesamt wäre es wünschenswert, die Befestigungsmittel aus einem Material fertigen zu können, das den folgenden Anforderungen genügt:
1. Mechanische Beanspruchbarkeit
   Die Fixierung eines Kopfes eines Patienten muss sehr stark und sehr sicher erfolgen. Die lokalen mechanischen Materialbeanspruchungen der Befestigungsmittel sind extrem hoch, da die mechanischen Kräfte zur Fixierung auf einen sehr kleinen Bereich auf die Spitze des Befestigungsmittels einwirken. Wenn hohe Kräfte auf das Befestigungsmittel wirken, darf es zum einen nicht zu einer kritischen Deformation des Befestigungsmittels kommen. Zum anderen darf das Befestigungsmittel nicht zerstört werden, es dürfen keine Absplitterungen erfolgen, da diese Teile nach dem Entfernen des Befestigungsmittels im Kopf des Patienten verbleiben könnten, was zu Verletzungen und Unverträglichkeiten führen könnte.
2. Sterilisierbarkeit
   Da das Befestigungsmittel als invasives Medizinprodukt eingesetzt werden soll, ist es notwendig, dass es sterilisiert werden kann. Dabei sind grundsätzlich verschiedene Sterilisationsarten möglich, z. B. Hitzesterilisationsverfahren (Dampf- und Heißluftsterilisation), Kaltsterilisationsverfahren (Gassterilisation und Sterilisation mit ionisierender Strahlung) und Sterilisation mit wässrigen Lösungen (Aldehyde, Peressigsäure, Halogene, Peroxide, etc.). Wird z.B. eine Sterilisation durch Hitze durchgeführt, so muss das zu sterilisierende Material eine gute Hitzebeständigkeit und eine geringe Wasserabsorptionsfähigkeit aufweisen. Bei chemischen Sterilisationen sind zwar geringere Temperaturbeständigkeiten erforderlich, dafür muss das Material aber chemisch inert sein, und der Sterilisationsvorgang dauert im allgemeinen wesentlich länger.
3. Biokompatibilität
   Bei dem Befestigungsmittel handelt es sich um eine invasiv-medizinische Vorrichtung, die für den kurzzeitigen Gebrauch geeignet ist (Klasse IIa gemäß Anhang IX der Richtlinie 93/42/ EWG). Die Befestigungsmittel durchstechen sowohl die Haut als auch den Knochen eines Patienten. Deshalb ist Biokompatibilität des Materials sehr wichtig.
4. Artefaktfreiheit bei bildgebenden Verfahren
   Viele Operationen, die mit höchster Präzision durchgeführt werden müssen, werden heutzutage durch bildgebende Verfahren begleitet. Das bedeutet, dass während der Operation eine Aufnahme (z.B. mittels eines CT-Scanners oder mittels eines MRT-Scanners) erfolgt. Dabei ist es wichtig, qualitativ hochwertige Aufnahmen zu erreichen. Störend sind bei diesen Aufnahmen insbesondere Artefakte, die durch die Verwendung von Befestigungsmitteln aus bestimmten Materialien hervorgerufen werden. Grundsätzlich gilt, dass insbesondere Metalle solche Artefakte hervorrufen; aber auch bei der Verwendung von Keramiken oder Kunststoff, die an sich strahlendurchlässig sind, werden solche Artefakte beobachtet. Verstärkt wird das Auftreten von Artefakten im Allgemeinen auch durch die Form der Artefakt-gebenden Gegenstände, z.B. durch Spitzen oder Kanten. Es ist praktisch nicht möglich, exakt vorherzusagen, ob bei der Verwendung eines bestimmten Befestigungsmittels Artefakte bei einem bildgebenden Verfahren auftreten werden oder nicht und wenn ja, in welcher Form und Stärke.

Die gängigen verwendeten Befestigungsmittel aus Metall, insbesondere aus Edelstahl und Titanlegierungen, sind zwar biokompatibel, verursachen aber zahlreiche Artefakte.

Es ist aus dem Stand der Technik bekannt, dass versucht wurde, einen Pin zur Verwendung in Kombination mit Kopfklemmen aus Saphir herzustellen, der strahlendurchlässig (englisch "radiolucent") ist. Dieser Pin ist auf dem Markt aber nicht erhältlich, da er anscheinend den mechanischen Anforderungen nicht standhält.

### Beschreibung der Erfindung

Es ist die Aufgabe der vorliegenden Erfindung, ein Befestigungsmittel zur Lagefixierung eines Körpers, insbesondere eines Kopfes, für medizinische Zwecke, insbesondere bei Operationen, bereitzustellen, der hinsichtlich der oben genannten Kriterien mechanische Beanspruchbarkeit, Sterilisierbarkeit, Biokompatibilität und Artefaktfreiheit bei bildgebenden Verfahren gegenüber dem Stand der Technik verbesserte Eigenschaften aufweist.

Die Aufgabe wird gelöst durch den Gegenstand der unabhängigen Ansprüche.

Abhängige Ansprüche sind auf bevorzugte Ausführungsformen der Erfindung gerichtet.

Ein erfindungsgemäßes Befestigungsmittel zur Lagefixierung eines Körpers, insbesondere eines Kopfes, für medizinische Zwecke, insbesondere bei Operationen, weist einen Halteabschnitt und einen Eindringabschnitt auf, der sich an den Halteabschnitt anschließt. Als Halteabschnitt des Befestigungsmittels wird dabei derjenige Abschnitt verstanden, der mit einer Fixierungsvarrichtung verbindbar ist. Im Allgemeinen handelt es sich dabei um einen zylinderförmigen Körper, der z.B. im Falle eines Pins für eine Kopfklemme an einem bestimmten Punkt in diese hineingesteckt und darin festgeschraubt wird. Der Halteabschnitt ist aber nicht zwangsweise zylinderförmig ausgebildet, andere Formen sind durchaus denkbar. Der Halteabschnitt könnte z.B. anstelle eines runden einen rechteckigen Querschnitt aufweisen. Der Eindringabschnitt, der sich an den Halteabschnitt anschließt, ist ein Abschnitt, der sich hin zu der Stelle, mit der das Befestigungsmittel in den Körper eindringt, verjüngt. Der Eindringabschnitt eines Befestigungsmittels dringt bei Verwendung des Befestigungsmittels wenigstens teilweise in den Körper ein. Ein solcher Eindringabschnitt ist z.B. kegelförmig ausgebildet. Halteabschnitt und Eindringabschnitt sind bevorzugt massiv, d.h., nicht hohl, ausgebildet.

Beispielhaft soll kurz ein herkömmlicher Pin betrachtet werden. Dieser ist einstückig ausgebildet, wobei seine gesamte Form sich aus eine zylindrischen Form und einer Kegelform zusammensetzt. Die zylindrische Form bildet in diesem Fall den Halteabschnitt, und der kegelförmige Abschnitt bildet den Eindringabschnitt.

Wenn Halteabschnitt und Eindringabschnitt einstückig ausgebildet sind, ist es auch möglich, dass Halteabschnitt und Eindringabschnitt kontinuierlich ineinander übergehen und eine scharfe Trennung zwischen Halteabschnitt und Eindringabschnitt nicht angebracht ist. Gleichwohl wird das Befestigungsmittel an einer Seite gehalten werden und an der anderen Seite in den Körper zur Lagefixierung eindringen. Insofern weist es auch dann einen Halteabschnitt und einen Eindringabschnitt auf.

Gemäß einer ersten Ausführungsform der Erfindung besteht das Befestigungsmittel wenigstens teilweise aus unsubstituiertem, einfach substituiertem oder mehrfach substituierten Polyphenylen, insbesondere aus unsubstituiertem, einfach oder mehrfach substituiertem Polyparaphenylen. Dabei kann das gesamte Befestigungsmittel einstückig aus den genannten Stoffen bestehen, es ist alternativ auch möglich, dass nur der Eindringabschnitt aus den genannten Materialien besteht. Der Halteabschnitt kann aus einem einzigen Abschnitt oder wiederum aus mehreren Abschnitten aufgebaut sein. Auch der Halteabschnitt kann ganz oder teilweise aus den oben angegebenen Materialien bestehen.

Gemäß einer bevorzugten Ausführungsform handelt es sich bei dem Polyphenylen um Polyparaphenylen. Die Struktur des Polyparaphenylen ist eine lineare Kette, die der Substanz eine besonders hohe Stabilität verleiht. Es ist aber auch möglich, ein Polyphenylen vorzusehen, indem die monomeren Phenyleinheiten einmalig, mehrmalig oder durchgehend in meta- und/oder ortho-Stellung verknüpft sind.

Zur einfachen oder mehrfachen Substitution der Polyphenylene, insbesondere der Polyparaphenylene, können insbesondere die nachfolgend aufgeführten Substituenten verwendet werden: C₁ bis C₈-Alkyle, d.h. Methyl-, Ethyl-, Propyl-, Butyl-, Pentyl-, Hexyl-, Heptyl- und/oder Oktyl-Gruppen. Die C₁-C₈-Alkylsubstituenten können insbesondere linear oder verzweigt sein. So sind ebenfalls iso-Propyl, sec-Butyl, tert-Butyl-Substituenten, sowie jegliche Isomere der C₅-C₈-Alkyl-Substituenten möglich. Des weiteren kann es sich bei den Substituenten um C₂ bis C₈-Alkenyle handeln; d.h. um gerade oder verzweigt verknüpfte Ethenyl-, Propenyl-, Butenyl-, Pentenyl-, Hexenyl-, Heptenyl- und/oder Oktenyl-Gruppen. Des weiteren kann es sich bei den Substituenten um gerade oder verzweigt verknüpfte C₂ bis C₈-Alkinyl-Gruppen handeln; d.h. um Ethinyl-, Propinyl-, Butinyl-, Pentinyl-, Hexinyl-, Heptinyl- und/oder Oktinyl-Gruppen, die jeweils gerade oder verzweigt verknüpft sind.

Darüber hinaus kann es sich bei den Substituenten um C₁-C₈-Alkoxyl, d.h. Methoxy-, Ethoxy-, Propoxy-, Butoxy-, Pentoxy-, Hexoxy-, Heptoxy und/oder Oktoxy-Gruppen handeln.

Auch im Falle der Alkenyl- und/oder der Akinylsubstituenten als auch der Alkoxysubstituenten können jegliche denkbaren isomeren Formen substituiert sein.

Auch können die Substituenten cyclische, nichtaromatische oder aromatische C₄-C₈-AlkylSubstituenten, wie beispielsweise cyclo-Butyl, cyclo-Pentyl, cyclo-Hexyl, cyclo-Heptyl und/oder cyclo Octylreste sein.

Gemäß einer bevorzugten Ausführungsform können ein oder mehrere Substituenten der Polyphenylene, insbesondere der Polyparaphenylene, aus der Hauptgruppe der Halogene gewählt werden, z.B. Chlor, Jod und/oder Brom. Des weiteren können die Substituenten aus einer Nitrogruppe, einer Hydroxylgruppe und/oder einer Aminogruppe bestehen bzw. diese Gruppen aufweisen.

Gemäß einer bevorzugten Ausführungform kann bei einfacher oder mehrfacher Substitution der Polyphenylene, insbesondere der Polyparaphenylene, ein Substituent eine Phenyl-, Benzyl-oder Benzoyl-Gruppe sein. Die Phenyl-, Benzyl- oder Benzoyl-Gruppe kann sich z.B. im Falle von Polyparaphenylen in orto- und/oder rrzeta-Stellung befinden.

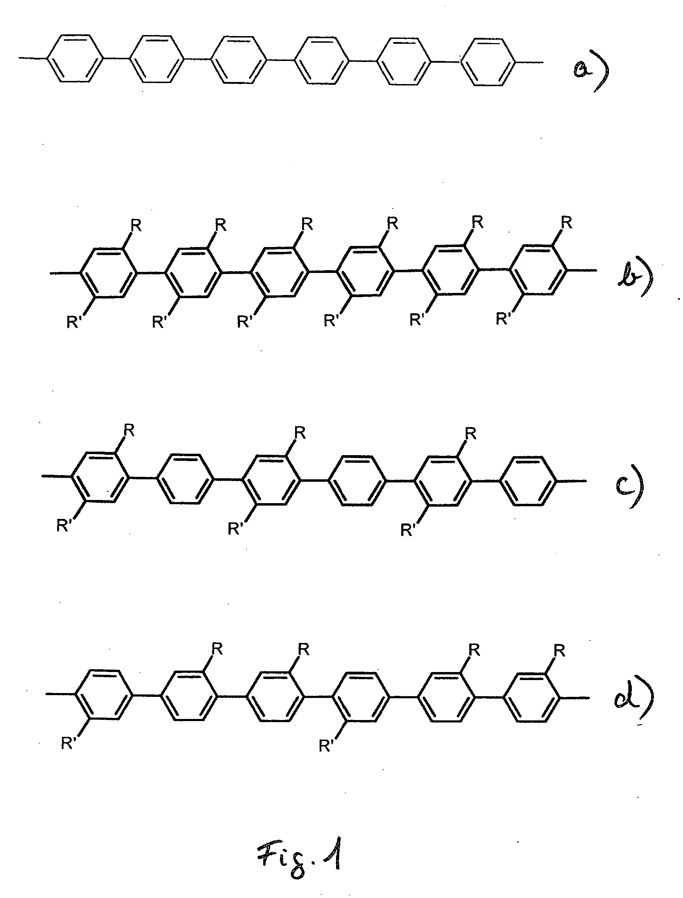

Die hier aufgeführten Substituenten sind beispielhaft in Figur 1 als R und R' bezeichnet.

Es ist möglich, dass alle vorgenannten unsubstituierten, einfach substituierten oder mehrfach substituierten Polyphenylene, insbesondere alle oben genannten unsubstituierten, einfach oder mehrfach substituierten Polyparaphenylene, faserverstärkt sind.

Gemäß einer bevorzugten Ausführungsform handelt es sich bei dem Material des Befestigungsmittels um unsubstituierte, einfach substituierte oder mehrfach substituierte Polyphenylen-Copolymere, insbesondere um unsubstituierte, einfach substituierte oder mehrfach substituierte Polyparaphenylen-Copolymere. Als Substituenten kommen jeweils alle vorstehend explizit aufgeführten Substituenten in Frage. Bei allen vorgenannten Copolymeren kann es sich um statistische Copolymere handeln, in denen die Verteilung von mindestens zwei verschiedenen Monomeren in der Kette zufällig ist. Es kann sich um Gradientcopoloymere handeln, in denen sich der Anteil eines Monomers im Vergleich zu mindestens einem weiteren Anteil des Monomers im Verlauf der Kette verändert. Es kann sich um ein alternierendes oder abwechselndes Copoloymer mit einer regelmäßigen Anordnung der mindestens zwei verschiedenen Monomeren entlang der Kette handeln. Es kann sich auch um Blockcopolymere handeln, welche aus mindestens zwei längeren Sequenzen oder Blöcken eines jeden Monomeres bestehen. Je nach der Anzahl der Blöcke spricht man auch von Biblock-, Triblock-, ..., C-Polymeren. Alternativ kann es sich um sog. Pfropfcopolymere handeln, bei denen die Blöcke eines Monomers auf das Gerüst (Rückgrat) eines anderen Monomers aufgepfropft sind.

Gemäß einer bevorzugten Ausführungsform besteht das Befestigungsmittel wenigstens teilweise aus einem Polyparaphenylen-Copolymer, wobei das erste Monomer einfach in ortho-Stellung substituiert ist, wobei das zweite Monomer einfach in meta-Stellung substituiert ist, und wobei der Substituent jeweils eine Benzoyl-Gruppe (-CO-C₆H₅) ist. Dabei sind das erste Monomer und das zweite Monomer bevorzugt alternierend angeordnet. Es ist aber auch möglich, dass es sich bei dem Polyparaphenylen-Copolymer um ein statistisches Copolymer, um ein Gradient-Copolymer, ein Block-Copolymer oder um ein Pfropf-Copolymer handelt.

Fig. 1 zeigt verschiedene Möglichkeiten, wie ein Polyphenylen gemäß der Erfindung ausgestaltet sein kann. Fig. 1a zeigt einen Ausschnitt aus einer unsubstituierten Kette von Polyparaphenylen. Gut erkennbar ist die lineare Struktur der Kette, die für die hohe Stabilität des Polyparaphenylens hauptverantwortlich ist. Fig. 1b zeigt einen Ausschnitt aus einer Kette von mehrfach substituiertem Polyparaphenylen. Jede Phenyl-Einheit ist mehrfach substituiert. Die Substituenten sind durch R bzw. R' angedeutet. Dabei können R und R' identisch oder verschieden sein. In dem Beispiel von Fig. 1b sind die Substituenten einander in Para-Stellung gegenüberliegend angeordnet, d.h., bezüglich der Kette aus Phenyl-Einheiten befinden sie sich jeweils in 2,5-Position. Fig. 1c zeigt einen weiteren Ausschnitt aus einer Kette von Polyparaphenylen, in der die einzelnen Phenyl-Einheiten abwechselnd unsubstituiert und mehrfach (in diesem Falle zweifach) substituiert vorliegen. Fig. 1d zeigt ein weiteres Beispiel für einen Kettenausschnitt eines Polyparaphenylens, wobei jede einzelne Phenyl-Einheit einfach substituiert ist. Die Substituenten befinden sich entweder in Ortho- oder in Meta-Stellung. Gemäß dem gezeigten Beispiel befindet sich ein Substituent R' in Ortho-Position, wohingegen sich ein Substituent R stets in Meta-Position befindet. Die Substituenten R und R' können verschieden sein, sie können aber auch gleichartig sein. Bei den Substituenten R und R' kann es sich um alle vorstehend explizit aufgeführten Substituenten handeln.

Gemäß einer weiteren bevorzugten Ausführungsform besteht das Befestigungsmittel wenigstens teilweise aus einer Siliziumnitrid-Keramik, insbesondere aus einer β-Siliziumnitrid-Keramik.

Dabei kann es sich um eine reine Siliziumnitrid-Keramik handeln. Es ist auch möglich, dass der Siliziumnitrid-Keramik andere Keramiken beigemischt bzw. beigefügt sind. Es ist auch möglich, dass es sich um eine faserverstärkte Siliziumnitrid-Keramik handelt. Bei einer reinen Siliziumnitrid-Keramik sind hohe Anforderungen an die Fertigung zu stellen, da selbst kleine Fertigungsfehler oder Kratzer auf der Oberfläche zum Startpunkt eines Risses werden können. Es kann deshalb zu einem spröden Bruchverhalten kommen. Durch die Verwendung von Langfasern zur Verstärkung der Keramik ist es möglich, den Risswiderstand drastisch zu erhöhen und zu verbesserten Eigenschaften wie z.B. erhöhter Dehnbarkeit, Bruchzähigkeit und hhermosehockbeständigkeit zu gelangen, was einen erweiterten Anwendungskreis von Siliziumnitrid-Keramiken und technischen Keramiken allgemein ermöglicht. Siliziumnitrid als ingenieur-keramischer Werkstoff zeichnet sich durch eine verhältnismäßige hohe Festigkeit aus.

Gemäß einer bevorzugten Ausführungsform ist bei dem erfindungsgemäßen Befestigungsmittel der Eindringabschnitt und/oder der Halteabschnitt achsenrotationssymmetrisch ausgebildet. Die Achse verläuft dabei in der Richtung, in die das Befestigungsmittel in den Körper für Lagefixierungszwecke eindringt. Bevorzugt ist der Halteabschnitt zylinderförmig ausgebildet. Bevorzugt ist der Eindringabschnitt kegelförmig ausgebildet. Es ist auch möglich, dass der Eindringabschnitt sich aus einem zylindrischen Abschnitt und einem kegelförmigen Abschnitt zusammensetzt. Dabei gehen die beiden verschiedenen geometrischen Formen bevorzugt ineinander über, und der Eindringabschnitt ist einstückig ausgebildet.

Gemäß einer bevorzugten Ausführungsform weist der Eindringabschnitt eine Spitze auf, und der gesamten Eindringabschnitt hat nicht die Form eines Kegels. Es kann dann davon gesprochen werden, dass der Eindringabschnitt eine Spitze aufweist, wenn sich der Eindringabschnitt zu einer Stelle, die zu vorderst in den zu fixierenden Körper eindringt, dermaßen verjüngt, dass das Eindringen in den Körper erfolgen kann. Die Spitze kann insofern nahezu idealisiert spitz sein, es ist aber auch möglich, dass die Spitze selbst noch einen messbaren Durchmesser aufweist, der geringer oder gleich 0,5 mm, bevorzugt geringer oder gleich 0,2 mm und äußerst bevorzugt geringer oder gleich 0,1 mm beträgt. In jedem Fall muss die Spitze spitz genug sein, um problemlos in den zu fixierenden Körper einzudringen.

Bevorzugt hat der gesamte Eindringabschnitt, wie oben bereits erwähnt, nicht die Form eines Kegels. Dieses Erfordernis ist bei jeglicher Form des Eindringabschnittes - und zwar bei Betrachtung des gesamten Eindringabschnittes - als erfüllt anzusehen, wenn die gesamte Form von der Form eines Kegels abweicht. Dabei ist es nicht als Abweichung zu betrachten, dass die Spitze, die der Eindringabschnitt aufweist, nicht als idealisierte Spitze vorliegt, sondern selbst - wie oben beschrieben, einen noch messbaren Durchmesser aufweist. Betrachtet wird vielmehr die äußere Form des gesamten Eindringabschnittes. Es ist z.B. möglich, dass ein erster Bereich des Eindringabschnittes sehr wohl die Form eines Kegels hat, sich darin aber ein weiterer Bereich des Eindringabschnittes anschließt, der nicht kegelförmig ist. Dann liegt insgesamt keine Kegelform des gesamten Eindringabschnittes vor. Der angesprochene zweite Bereich des Eindringabschnittes kann z.B. ogivenförmig ausgebildet sein. Es ist auch möglich, dass es sich um eine konkaven oder konvexen Abschnitt handelt, der sich an den kegelförmigen Abschnitt anschließt, der wiederum - wenn der Kegel spitz zuläuft - eine Spitze aufweist.

Gemäß einer bevorzugten Ausführungsform des Befestigungsmittels weist der Eindringabschnitt einen ersten Bereich in Form eines Kegelstumpfes, der an den Halteabschnitt angrenzt, und einen zweiten Bereich in Form eines Kegels, der die Spitze bildet. Dabei unterscheidet sich der Winkel des Kegelstumpfmantels bezüglich der Kegelstumpfachse von dem Winkel des Kegelmantels bezüglich der Kegelachse. Gemäß einer bevorzugten Ausführungsform setzt sich der gesamten Eindringabschnitt aus einem Kegel und einem Kegelstumpf zusammen. Dabei ist der so zusammengesetzte Eindringabschnitt bevorzugt einstückig ausgebildet. Bevorzugt ist es so, dass der Winkel des Kegelmantels bezüglich der Kegelachse größer ist als der angesprochene Winkel des Kegelstumpfmantels bezüglich der Kegelstumpfachse. Bevorzugt setzt sich der Eindringabschnitt aus einem Kegelstumpf und einem sich daran anschließenden Kegel zusammen, wobei der Kegelstumpf an den Halteabschnitt angrenzt bzw. sich daran anschließt. Es ist aber auch möglich, dass sich der Eindringabschnitt aus mehreren aufeinander folgenden Kegelstümpfen und einem Kegel, der wiederum die Spitze bildet, zusammensetzt. Dabei ist es bevorzugt so, dass der gesamte Eindringabschnitt einstückig ausgebildet ist. Bevorzugt ist der Eindringabschnitt auch mit dem Halteabschnitt einstückig ausgebildet.

Gemäß einer bevorzugten Ausführungsform ist es so, dass der Durchmesser des Befestigungsmittels, orthogonal zur Rotationsachse gemessen, von der Spitze hin zum Ende des Halteabschnittes, der nicht dem Eindringabschnitt zugewandt ist, monoton zunimmt. Dabei ist es bevorzugt so, dass der Durchmesser des Eindringabschnittes gemessen in einer Richtung orthogonal zur Achse, beginnend von dem Ende, das zwecks Lagefixierung eingerichtet ist, in einen Körper einzudringen, z.B. in Form einer Spitze, hin zu dem Ende, der sich an den Halteabschnitt anschließt, streng monoton zunimmt.

Gemäß einer bevorzugten Ausführungsform ist der Eindringabschnitt zweistückig ausgebildet, wobei das eine Stück mit dem anderen Stück in festem Kontakt ist, und wobei das eine Stück eine Spitze aufweist. Unter dem Begriff zweistückig wird dabei das Vorliegen von zwei separaten Werkstücken verstanden, die miteinander in festen Kontakt bringbar sind.

Fester Kontakt in diesem Zusammenhang bedeutet, dass der Kontakt fest genug ist, damit sich der Eindringabschnitt bei der Befestigung zwecks Lagefixierung eines Körpers und bei Wiederherausnahme nicht in seine zwei Bestandteile zerlegt. Der feste Kontakt kann z.B. durch Pressen oder Kleben herbeigeführt werden. Die Zweistückigkeit hat den Vorteil, dass es im Prinzip möglich ist, für jedes Stück des Eindringabschnittes ein anderes Material auszuwählen, das für seinen konkreten Einsatzort - d.h. für seine Position innerhalb des Eindringabschnittes und die damit verbundene Funktion - von Vorteil ist. Es ist auf diese Weise möglich, für die Spitze z.B. ein besonders mechanisch stabiles und festes Material auszuwählen, und für den übrigen Eindringabschnitt ein nicht ganz mechanisch so belastbares bzw. nicht so festes Material auszuwählen, das aber beispielsweise besser strahlendurchlässig ist.

Gemäß einer bevorzugten Ausführungsform umfasst das eine Stück des Eindringabschnittes eine Spitze aus Keramik, insbesondere eine Spitze aus einer Siliziumnitrid-Keramik. Das andere Stück ist bevorzugt aus unsubstituiertem, einfach substituiertem oder mehrfach substituiertem Polyphenylen, insbesondere aus unsubstituiertem, einfach oder mehrfach substituiertem Polyparaphenylen. Äußerst bevorzugt besteht das andere Stück aus einem Polyparaphenylen-Copolymer. Für die Polyphenylene, Polyparaphenylene und Polyparaphenylene-Copolymere gilt das bereits oben Ausgesagte. Alle dort genannten Konfigurationen und Substituenten sind geeignet.

Aufgrund der guten stofflichen Eigenschaften der oben beschriebenen unsubstituierten, einfach substituierten oder mehrfach substituierten Polyphenylene, insbesondere der unsubstituierten, einfach oder mehrfach substituierten Polyparaphenylene, und insbesondere der oben beschriebenen Polyparaphenylen-Copolymere, hinsichtlich Strahlendurchlässigkeit und mechanischer Beanspruchbarkeit/Härte sowie der vorhandenen Biokompatibilität und Sterilisierbarkeit erscheint es aussichtsreich, auch andere Gegenstände, die im Bereich der Implantat-Medizin verwendet werden, aus diesen Materialien herzustellen. Es erscheint vorteilhaft, Schrauben, Implantate, Osteosyntheseplatten, Nägel usw. aus den genannten Materialien zu fertigen. Auch könnte bei Operationen, während denen z. B. CT-Scans oder MRT-Scans durchgeführt werden, das gesamte Instrumentarium, das aus den beschriebenen unsubstituierten, einfach substituierten oder mehrfach substituierten Polyparaphenylenen gefertigt ist, während der Scans im Körper des Patienten verbleiben. Zu diesem Instrumentarium zählen beispielsweise Wundhaken, Retraktoren, Spreizer, Pinzetten, Klemmen, Fasszangen und Stapler für Klammernahtgeräte.

Die Erfindung wird noch besser verstanden werden in Zusammenhang mit den im Folgenden beschriebenen Figuren und Tabellen. Dabei zeigen:
- Fig. 1: zeigt verschiedene Beispiele für unsubstituiertes, einfach bzw. mehrfach substituiertes Polyparaphenylen;
- Fig. 2: zeigt an einem Modell, wie ein Schädel mittels einer Kopfklemme, die Pins aufweist, fixiert wird;
- Fig. 3: zeigt einen Pin mit Markierungsringen;
- Fig. 4: zeigt schematisch einen Versuchsaufbau zur Messung der auf die Pins wirkenden Kräfte;
- Fig. 5: ist eine schematische Darstellung eines Pins;
- Fig. 6A bis 6H: zeigen Diagramme mit Messwerten, in denen die einwirkenden Kräfte über der Zeit für verschiedene Pins und verschiedene Materialien der Pins aufgetragen sind;
- Fig. 7: zeigt verschiedene Pins aus verschiedenen Materialien nach Benutzung in einer Kopfklemme;
- Fig. 8: zeigt verschiedene Röntgenschnittaufnahmen eines mit Gel gefüllten Schädelphantoms, wobei jeweils Pins aus verschiedenen Materialien am Schädel fixiert sind;
- Fig. 9A: zeigt schematisch die Geometrie eines Pins;
- Fig. 9B und 9C: zeigen Abbildungen entsprechender Pins aus verschiedenen Materialien;
- Fig. 10A und 10B: zeigen schematische Darstellung eines Pins, bei dem ein Eindringabschnitt zweistückig ausgebildet ist;
- Fig. 10C: zeigt eine Abbildung eines entsprechenden Pins;
- Fig. 11: zeigt verschiedene Röntgenschnittaufhahmen eines mit Gel gefüllten Schädelphantoms, wobei Pins aus verschiedenen Materialien am Schädel befestigt sind;
- Fig. 12: zeigt verschiedene Pins nach Verwendung in einer Kopfklemme, wobei die dargestellten Pins die Geometrie aufweisen, die in Fig. 9A dargestellt ist;
- Fig.13: zeigt einen Versuchsaufbau zum Testen der maximalen axialen Belastung;
- Fig. 14: zeigt verschiedene Pins nach dem maximalen Belastungstest, der mit der Anordnung, die in Fig. 13 gezeigt ist, durchgeführt wurde;
- Fig. 15: zeigt einen Versuchsaufbau, mit dem die maximale Krafteinwirkung in Kombination mit Biegemomenten simuliert und gemessen wurde;
- Fig. 16: zeigt Pins aus verschiedenen Materialien nach der Durchführung des Tests mittels der in Fig. 15 gezeigten Anordnung;
- Fig. 17: zeigt ein Diagramm, in dem die einwirkenden Kräfte auf verschiedene Pins, die aus Siliziumnitrid bestehen, über der Zeit aufgetragen sind, wobei die in Fig. 9A dargestellte Geometrie für die Pins verwendet wurde; und
- Fig. 18: zeigt ein Diagramm, in dem die einwirkenden Kräfte auf verschiedene Pins über der Zeit aufgetragen dargestellt werden, wobei die verwendeten Pins aus TECAMAX SRP in Kombination mit SNI 750 bestehen und die Geometrie diejenige ist, die in den Fig. 10A und 10B dargestellt ist.

Es wurden mehrere Testserien durchgeführt, um Befestigungsmittel zu ermitteln, die den oben genannten Kriterien mechanische Beanspruchbarkeit, Sterilisierbarkeit, Biokompatibilität und Artefaktfreiheit bei bildgebenden Verfahren genügen. Dabei wurden Materialien aus der Gruppe der Keramiken und aus der Gruppe der Polymere ausgewählt und getestet. Es handelte sich dabei um die folgenden Materialien:
Keramiken:
   - MACOR (Mischung aus verschiedenen Keramiken)
   - SNI 750 (Siliziumnitrid)
   - ZrO₂ (Zirkoniumoxid)
Polymere:
   - TECAMAX SRP (selbstverstärkendes Polyparaphenylen-Copolymer mit Benzoyl-Substituenten; vgl. Anspruch 3)
   - TECAPEEK classix (Polyetheretherketon)
   - TECAPEEK CF30 (Polyetheretherketon mit 30 % Kohlenstofffasern)
   - TECAPEEK GF30 (Polyetheretherketon mit 30 % Glasfasern)

Die Untersuchungen hinsichtlich Biokompatibilität erfolgten in Form von einer Literaturrecherche. MACOR ist biokompatibel und wird bereits in der Hals-Nasen-Ohrenheilkunde als Gehörknöchelchenimplantat benutzt.

SNI 750 wurde in in-vitro-Tests untersucht, und es wurde herausgefunden, dass Siliziumnitrid sich bio-inert verhält. Allerdings liegen für das SNI 750 von der "Ceratec GmbH", deren SNI 750 für die folgenden Test verwendet wurde, noch keine Zertifikate über die Biokompatibilität dieses Materials vor.

Zirkoniumoxid wird häufig bei Operationen verwendet, z.B. für Hüftimplantate. Umfassende in-vüro- und in-vivo-Tests haben gezeigt, dass sich Zirkoniumoxid bio-inert verhält.

TECAMAX SRP der Firma "Ensinger GmbH" ist biokompatibel, und es liegen die erforderlichen Zertifikate hierüber vor.

Betreffend TECAPEEK classix wurden Untersuchungen in Osteoblast-Kulturen durchgeführt, die zeigen, dass TECAPEEK classix kein zelltoxisches Verhalten zeigt. Zylindrische Proben von PEEK wurden in Muskelgewebe von Mäusen akzeptiert, und zwar nach einer Implantations-Periode von 12 Wochen.

Bei TECAPEEK CF30 und TECAPEEK GF30 handelt es sich um faserverstärkte Polymere, welche hinsichtlich ihrer Biokompatibilität kritischer sind, da sie eine geringere chemische Beständigkeit besitzen und eine Tendenz dahingehend besteht, dass sie Teilchenanordnungen bilden. Eine Gewebeinfektion wäre in diesem Fall die unerwünschte Folge.

Eine EO-Sterilisation kann für alle untersuchten Materialien verwendet werden.

Um die mechanische Belastbarkeit des Materials und seine Eignung zur Fixierung eines Körpers während Operationen zu untersuchen, wurden verschiedene Tests durchgeführt. Bei diesen Tests handelte es sich zum einen um Kräftemessungen, wobei mit verschiedenen Sensoren gemessen wurde, welche Kräfte bei der Fixierung des menschlichen Schädels auf den jeweiligen Pin einwirken. Des weiteren wurde die Eindringtiefe von konventionell erhältlichen Pins in den Kopf eines Patienten untersucht. Pins aus verschiedenen Materialien wurden nach ihrer Verwendung zur Lagefixierung eines Körpers dahingehend untersucht, ob sie Verformungen oder Defekte aufweisen.

Um die Artefaktbildung bzw. Artefaktfreiheit bei bildgebenden Verfahren zu untersuchen, wurden beispielhaft 3D CT-Scans durchgeführt, wobei Pins, die aus verschiedenen Materialien hergestellt waren, an einem speziellen mit Wachs-Gel gefüllten Kopf-Modell, dass das menschliche Gehirn simuliert, befestigt waren.

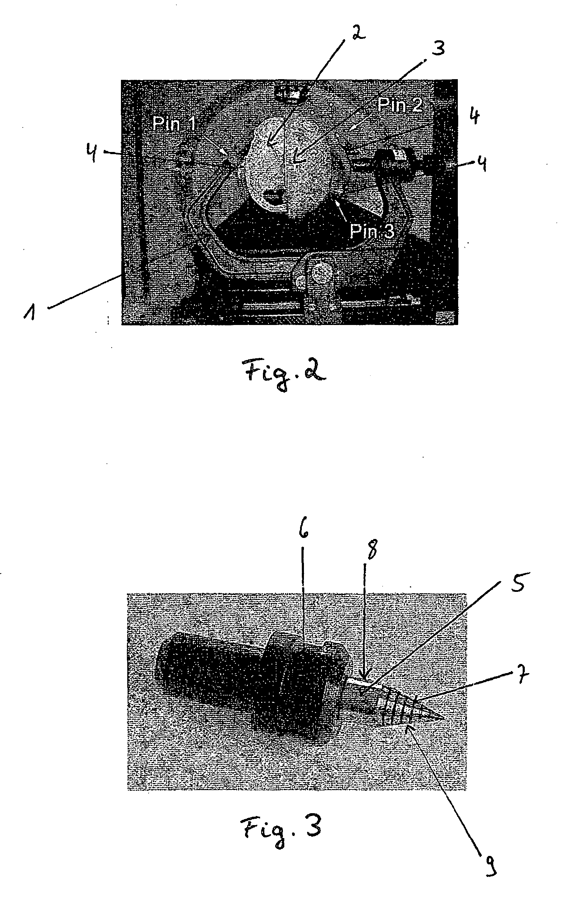

Figur 2 zeigt eine Kopfklemme, wie sie aus dem Stand der Technik bekannt ist, vom so genannten Mayfield-Typ. Die Kopfklemme 1 ist an drei Punkten mit Einfassungselementen 4 versehen, in denen sich jeweils fixiert ein so genannter Pin befindet. Dabei befindet sich Pin 1 relativ zentral auf der linken Seite des Kopfmodells 3, wohingegen sich Pin 2 und Pin 3 auf der anderen Seite des dargestellten Modells des Kopfes 3 befinden. Zur Veranschaulichung ist eine Abdeckung des Modells auf der linken Seite des Bildes weggelassen worden, und es wird in dem Modell das Gehirn 2 sichtbar. Bei den meisten erhältlichen Kopfklemmen handelt es sich um diese Form der Drei-Punkt-Kopfklemmen. Es sind aber auch Vier-Punkt-Kopfklemmen erhältlich.

Figur 3 zeigt einen Pin, der in Zusammenhang mit der Kopfklemme, die in Figur 2 dargestellt ist, verwendet werden kann, in Großaufnahme. Der Pin 5 ist in diesem Fall - wie im Stand der Technik üblich - aus Metall hergestellt. Er ist mit einem Haltemittel 6 fest in Verbindung. Der Pin weist einen Halteabschnitt 8 auf, der mit dem Haltemittel 6 in Verbindung steht, sowie einen Eindringabschnitt 9. Dieser Eindringabschnitt ist kegelförmig ausgebildet und wurde mit Ringen 7 markiert. Diese Ringe ermöglichen es, zu messen, wie weit der Pin 5 im Rahmen der verschiedenen Testreihen in den fixierten Körper bzw. den fixierten Kopf eingedrungen ist bzw. darin eindringt. Bei dem gezeigten mit Markierungsringen 7 versehenen Pin 5 handelt es sich um einen Pin aus Edelstahl.

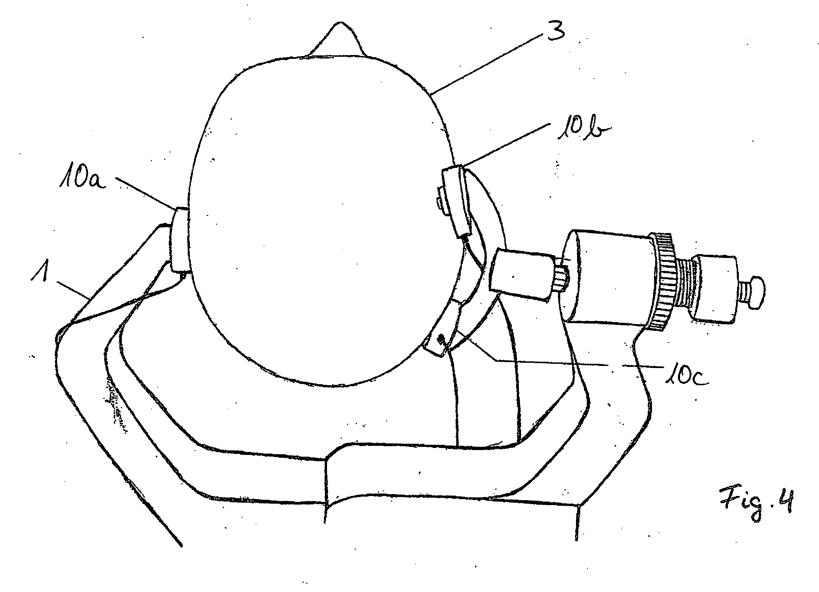

Figur 4 zeigt schematisch die Versuchsanordnung, die zur Messung der auf die Pins einwirkenden Kräfte verwendet worden ist. Gezeigt ist zunächst die Drei-Punkt-Kopfklemme 1. In diese ist ein Schädel 3 mittels drei verschiedener Pins eingefasst. An den Pins befinden sich Sensoren 10a, 10b und 10c, die jeweils die einwirkenden Kräfte auf die Pins 1, 2, 3 messen und die Daten an eine Datenauswerteeinheit (nicht gezeigt) weiterleiten.

### 1. Versuchsreihe

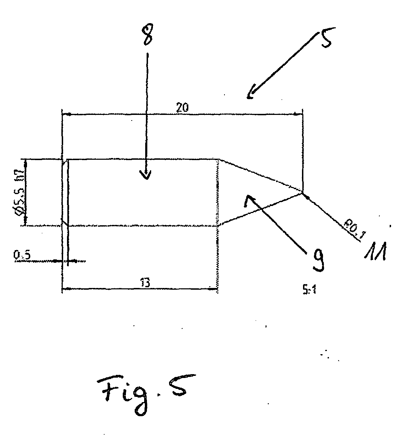

In Figur 5 ist schematisch die Geometrie eines Pins 5 gezeigt, wie sie in einer ersten Versuchsreihe verwendet worden ist. Der Pin 5 ist einstückig ausgebildet und weist einen Halteabschnitt 8 und einen Eindringabschnitt 9 auf. Der Halteabschnitt ist zylinderförmig ausgebildet (dargestellt ist eine Schnittzeichnung entlang der Achse des Pins), und der Eindringabschnitt 9 ist kegelförmig ausgebildet. Der Eindringabschnitt 9 schließt sich an den Halteabschnitt 8 an und weist eine Spitze 11 auf. Diese Spitze ist keine perfekte Spitze, sondern eine Spitze, die selbst noch einen messbaren Durchmesser aufweist. Im vorliegenden Beispiel beträgt dieser 0,1 mm. Im dargestellten Beispiel der Figur 5 beträgt die gesamte axiale Länge des Pins 5 20 mm, wobei 13 mm davon auf den Halteabschnitt 8 entfallen. Im Allgemeinen ist der Halteabschnitt 8 länger als der Eindringabschnitt 9, dies ist jedoch nicht zwingend der Fall, sondern hängt davon ab, auf welche Art und Weise der Halteabschnitt mit einem geeigneten Haltemittel z. B. einer Kopfklemme befestigt werden soll. Die in Figur 5 gezeigte Dimensionierung ist somit nicht einschränkend, sondern nur beispielhaft zu verstehen. In einer ersten Messreihe wurde nun mittels des in Figur 3 dargestellten Pins 5 ermittelt, wie weit die Pins 1, 2 bzw. 3 im Rahmen der Lagefixierung eines Körpers bzw. eines Kopfes in den Kopf eindringen. Dazu wurde abgelesen, wie viele Ringe 9 nach abgeschlossenem Fixierungsprozess in den Kopf eingedrungen waren und nicht mehr zu erkennen waren. Dabei zeigte sich erwartungsgemäß, dass das Eindringverhalten für Pin 1, wie in Figur 2 dargestellt, ein anderes ist als für die Pins 2 und 3, wie in Figur 2 dargestellt. Dies ist die logische Konsequenz aus der Tatsache, dass auf der einen Seite des Kopfes, der in Figur 2 modellhaft gezeigt ist, ein einzelner Pin die gesamte Fixierungsleistung erbringen muss, während sich die einwirkenden Kräfte auf der anderen Seite auf die Pins 2 und 3 verteilen. Dabei wurde betreffend die Eindringtiefe Folgendes festgestellt:
Pin 1: Eindringtiefe < 1,5 mm (axiale Kraft ≈ 360 N)
Pin 2: Eindringtiefe ≤ 1,0 mm (axiale Kraft ≈ 250 N)
Pin 3: Eindringtiefe: < 1,0 mm (axiale Kraft ≈ 180 N).

Nachdem mit dem Pin 5, der mit Ringen 7 gekennzeichnet ist, die Eindringtiefe prinzipiell gemessen worden ist, wurden Pins aus verschiedenen Materialien für Testreihen herangezogen. Bei diesem Materialien handelte es sich um MACOR, SNI 750, TECAMAX SRP, TECAPEEK classix, TECAPEEK CF30 und TECAPEEK GF30. Hierzu wurden in einem Institut für Pathologie an zehn Leichen jeweils drei Versuchsreihen durchgeführt. Das Fixieren der Köpfe der Leichen wurde dabei von Neurochirurgen vorgenommen, die normaler Weise eine solche Fixierung bei zu behandelnden Patienten während Kopfoperationen vornehmen müssen, um eine möglichst realistische Testsituation herbeizuführen. Dabei wurde zunächst der Kopf der Leichen fixiert. Anschließend wurde die Fixierung getestet, indem versucht wurde, den Kopf zu bewegen. Danach wurde die Kopfklemme wieder gelockert und der Kopf der Leichen freigegeben. Die verwendeten Pins unterschiedlichen Materials wurden anschließend eingehender untersucht.

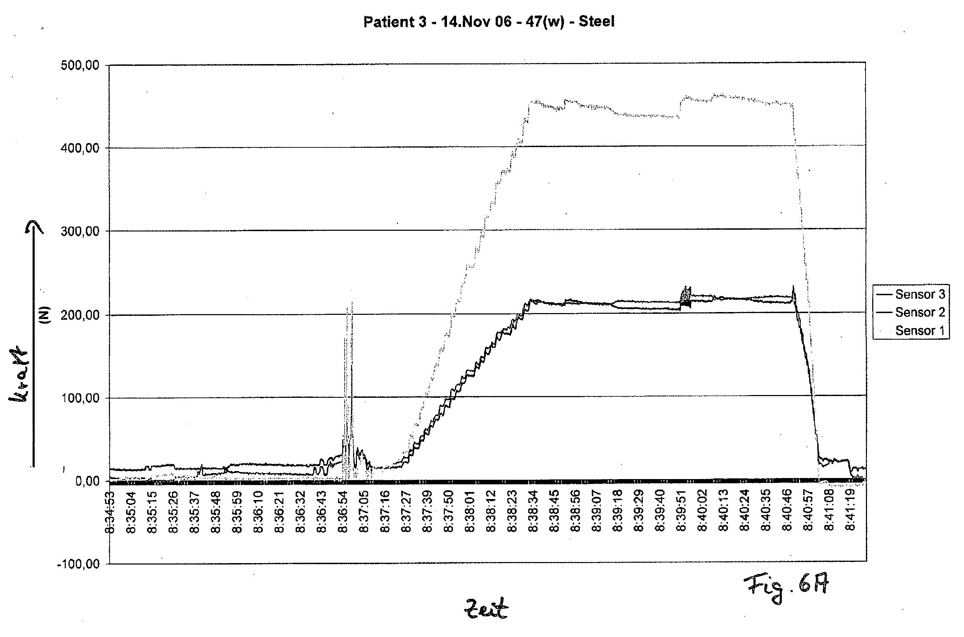

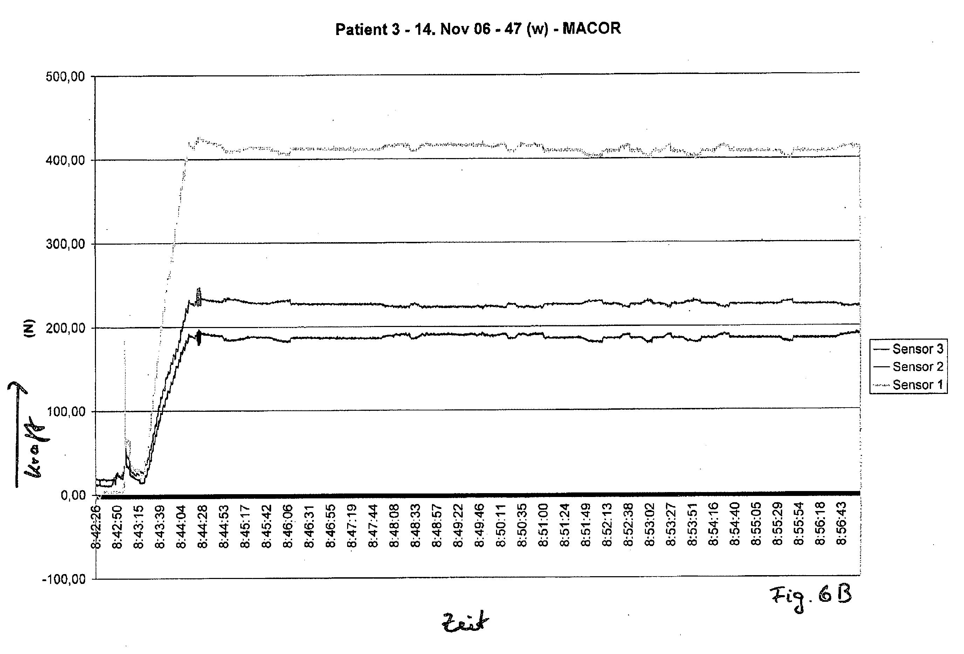

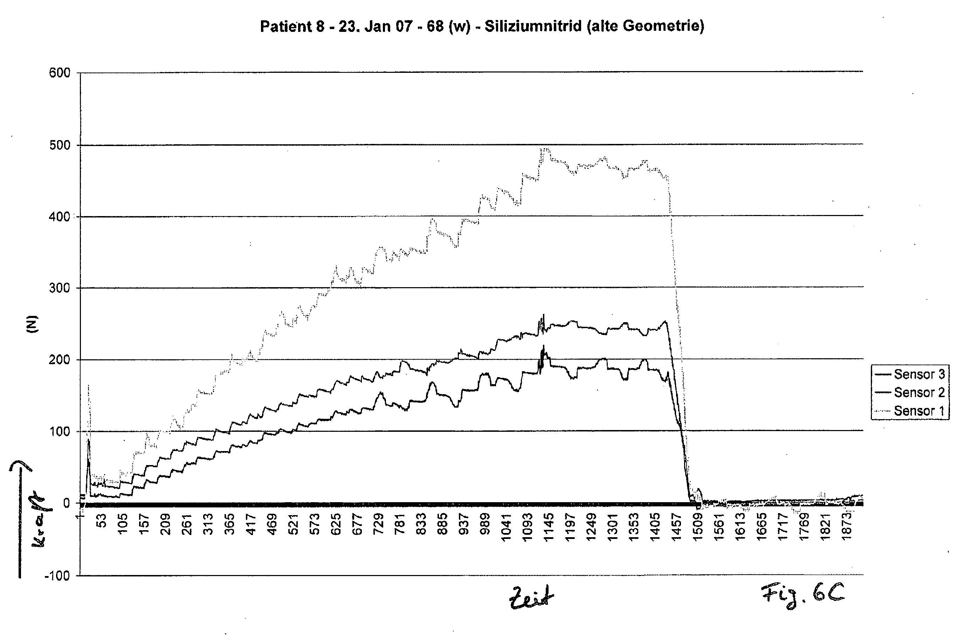

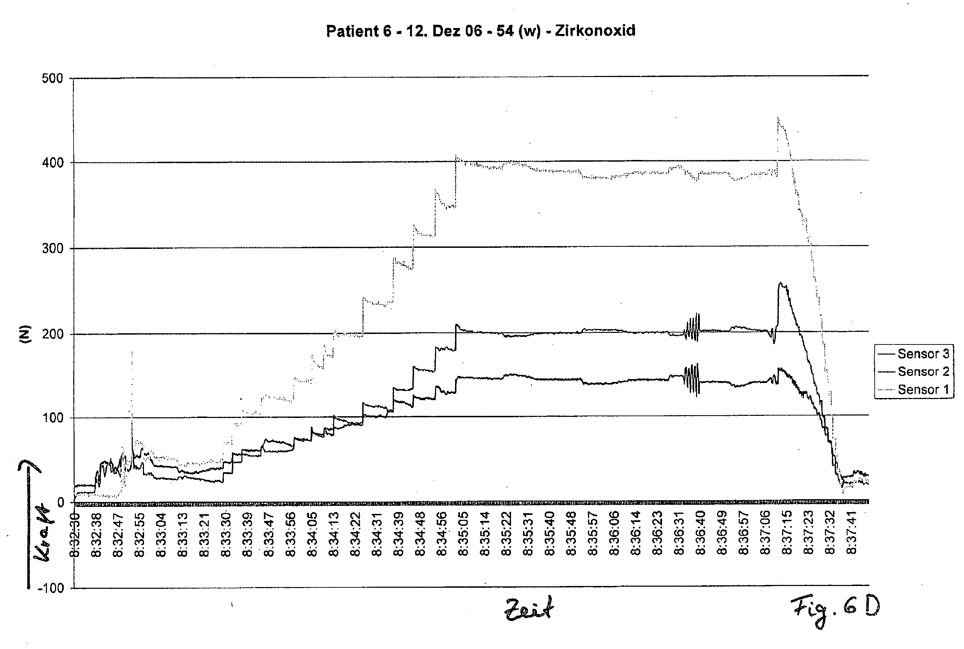

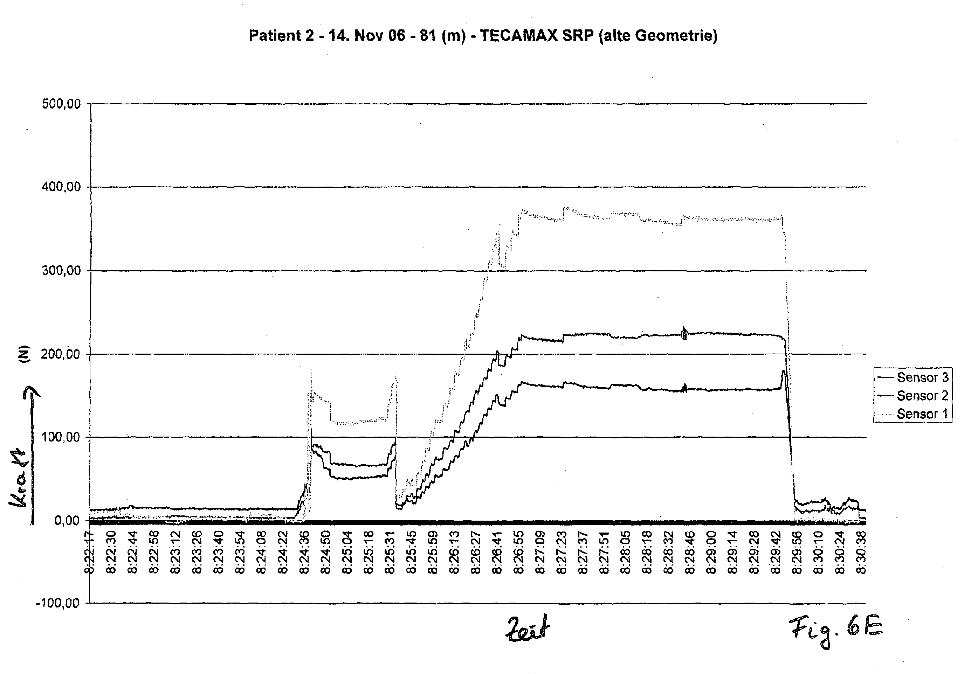

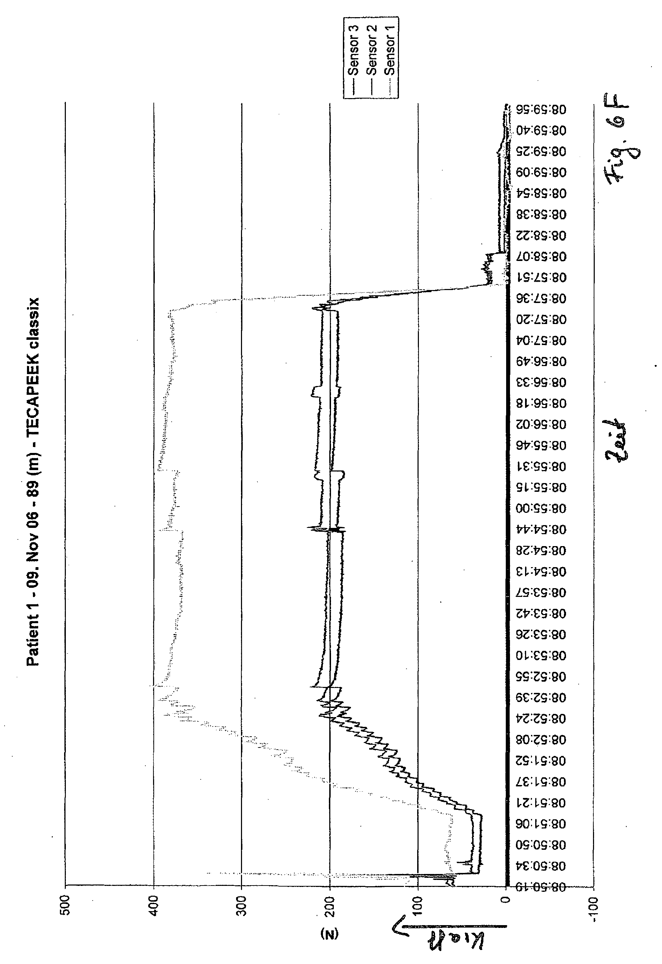

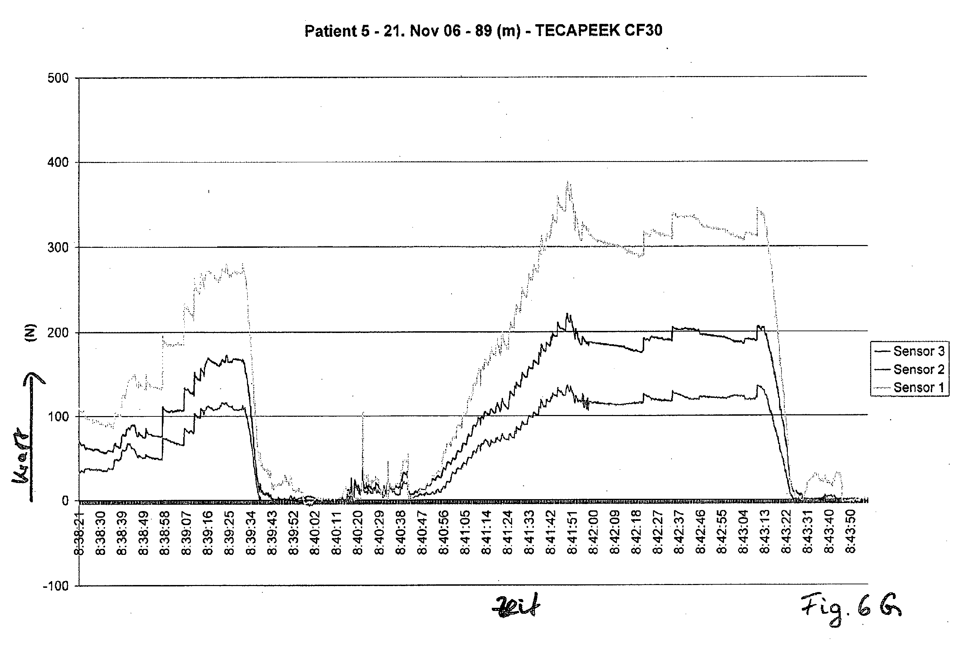

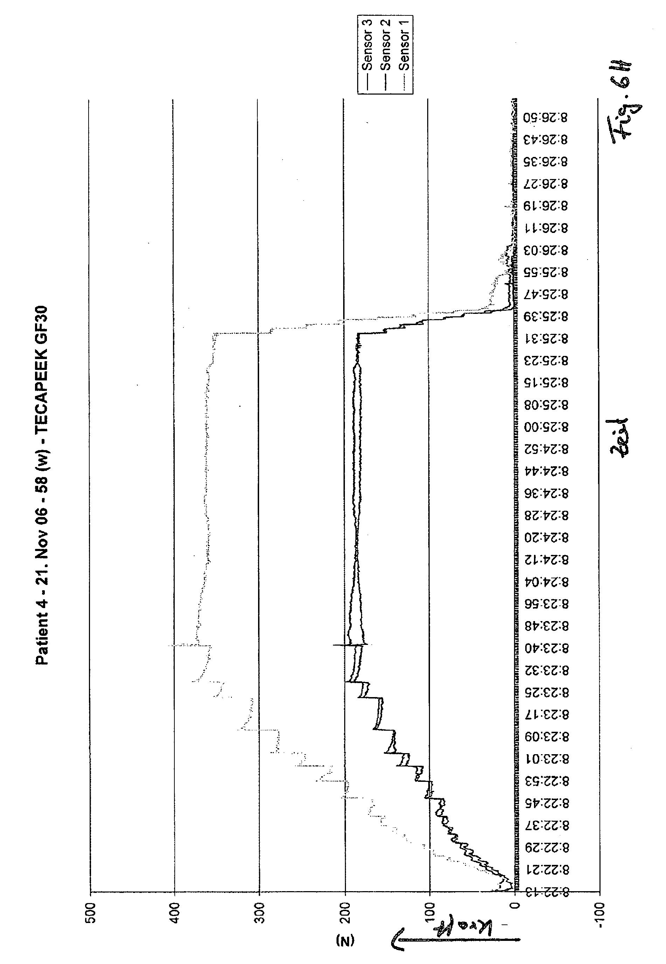

In den Figuren 6A bis 6H sind die mittels der Sensoren 1 bis 3 gemessenen Kräfte über der Zeit aufgetragen, die auf die Pins 1 bis 3 eingewirkt haben. Beispielhaft ist jeweils ein Diagramm pro verwendetem Pin-Maierial der Anmeldung beigefügt. Die einwirkenden Kräfte bewegen sich unabhängig vom verwendeten Pin-Material jeweils in einem gewissen Bereich. Die aus Pin 1 wirkenden Maximalkräfte befinden sich etwa im Bereich von 400 N. Es gibt gewisse Schwankungen um diesen Maximalwert herum, in einigen Fällen betrugen die Maximalwerte bei vollständiger Fixierung der Testperson circa 450 N (siehe Figur 6A; Stahl-Pin). In einem anderen Fall lagen die Maximalkräfte, die auf Pin 1 wirkten leicht unter den 400 N bei circa 380 N (siehe z. B. Figur 6B; TECAMAX SRP).

Die auf die Pins 2 und 3 einwirkenden Maximalkräfte waren deutlich geringer, da sich die Pins 2 und 3 auf derselben Seite der Kopfklemme befanden und - anders als bei Pin 1 - zwei Pins ein und dieselbe Kopfseite zu fixieren hatten. Die Maximalkräfte für Pins 2 und 3 lagen meist im Bereich von circa 200N, in der Tendenz war die auf Pin 3 einwirkende Maximalkraft leicht geringer als die auf Pin 2 einwirkende Maximalkraft.

Allen Kraftkurven, die den verschiedenen Pins zuzuordnen sind, ist gemeinsam, dass während des Fixierungsvorganges ein Anstieg der gemessenen Kraft erfolgt, der von einzelnen Mikro-Schwankungen in den Graphen abgesehen annähernd linear erfolgt. An diesen Anstieg schließt sich dann ein plateauförmiger Bereich der Graphen an, in dem die maximale Krafteinwirkung zu verzeichnen war.

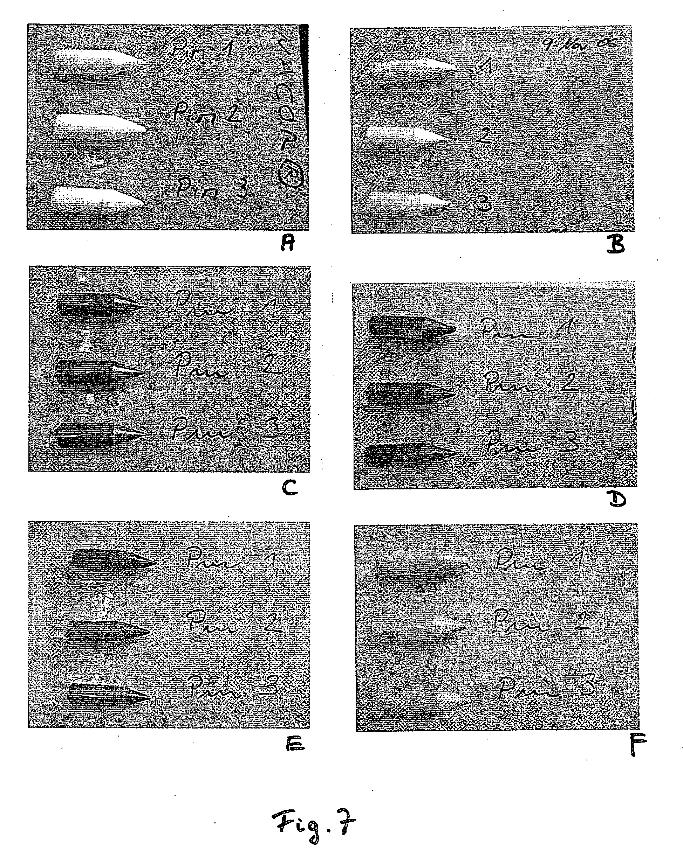

In Figur 7 sind exemplarisch jeweils die drei Pins eines Messvorganges abgebildet, die jeweils die Pins 1 bis 3 nach ihrer Verwendung in einer Kopfklemme, d. h. nach Fixierung eines Kopfes, zeigen. Die abgebildeten Pins bestehen aus den Folgenden Materialien:
A: MACOR; B: TECAPEEK classix; C: SNI 750; D: TECAPEEK CF30; E: TECAMAX SRP; F: TECAPEEK GF30. Die Pins wurden nun visuell mittels eines OP-Mikroskops hinsichtlich Deformationen und hinsichtlich aufgetretenen Defekten (Risse oder Absplitterungen) untersucht. Dabei ist zu erkennen, dass bei jeder Gruppe von Pins, die aus einem bestimmten Material besteht, Defekte aufgetreten sind. Diese sind allerdings unterschiedlich stark ausgeprägt. In Figur 7 ist zu erkennen, dass gelegentlich die Spitze eines Pins nach Verwendung fehlt. Häufig weisen die Pins auch Verformungen besonders im Bereich der Spitze auf, diese sind besonders stark ausgeprägt bei den Materialien TECAPEEK classix, TECAPEEK CF30 und TECAPEEK GF30. Die besten Ergebnisse wurden für die Pins aus den Materialien TECAMAX SRP und SNI 750 erhalten (vergleiche Figuren 7E und 7C). Die Pins haben nach Benutzung noch annähernd ihre symmetrische Idealform, die Spitze blieb weitgehend erhalten und wurde nur bei Pin 1, der jeweils den stärksten Belastungen ausgesetzt war, deformiert bzw. wies einen Defekt auf.

Die Ergebnisse dieses Material-Belastungstests sind in der folgenden Tabelle 1 zusammenfassend dargestellt:

**Tabelle 1**

| Material | Deformationen | Defekte |
|---|---|---|
| MACOR | - | - |
| SNI 750 | + | - |
| TECAMAX SRP | + | - |
| TECAPEEK classix | - | - |
| TECAPEEK CF30 | - | - |
| TECAPEEK GF30 | - | - |

In der Tabelle bedeutet ein "+"-Zeichen, dass ein taugliches Resultat erzielt wurde, d. h. SNI 750 und TECAMAX SRP weisen die geringsten Deformationen auf. Ein "-"-Zeichen in der Tabelle bedeutet, dass eine nicht so gute oder negative Tauglichkeit der Pins herausgefunden wurde. Des Weiteren beideutet "0", dass ein zufrieden stellendes Resultat erhalten wurde, und "++", dass ein hervorragendes Resultat erhalten wurde. Diese Notation gilt auch für alle folgenden Tabellen.

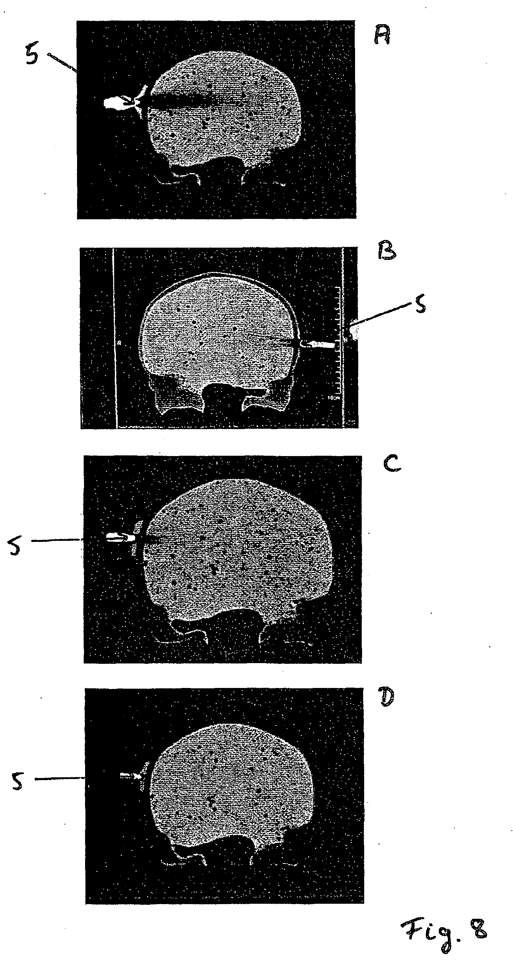

In einer nächsten Testreihe wurden die beiden hinsichtlich der mechanischen Belastbarkeit aussichtsreichsten Kandidaten für einen Pin hinsichtlich der gewünschten Strahlendurchlässigkeit und hinsichtlich ihrer Artefaktbildung untersucht. Dazu wurden verschiedene 3D-CT-Scanns mit Pins unterschiedlicher Materialien durchgeführt. Diese wurden an einem Modell befestigt, das das menschliche Gehirn simuliert (dabei handelt es sich um ein spezielles mit Wachs-Gel gefülltes Modell). Neben den beiden aussichtsreichsten Kandidaten SNI 750 und TECAMAX SRP wurden zum Vergleich Pins aus einer Titanlegierung und aus Zirkoniumoxid verwendet. Auf diese Weise enthaltene CT-Schnitt-Aufnahmen sind in Figuren 8A bis 8D dargestellt. Bei Figur 8A wurde ein Pin aus Titanium, bei Figur 8B ein Pin aus Zirkoniumoxid, bei Figur 8C ein Pin aus SNI 750 und bei Figur 8D ein Pin aus TECAMAX SRP verwendet. Die Pins sind in den Aufnahmen jeweils als weißer Bereich zu erkennen. Von den Pins 5 gehen bei Artefaktverursachung durch diese Pins schwarze Linien zumeist fächerförmig aus. Bei diesen schwarzen Linien handelt es sich um die vorher angesprochenen Artefakte, die eine genaue Analyse, der Aufnahme erschweren, was es zu vermeiden gilt. Es ist zu erkennen, dass bei den herkömmlicherweise verwendeten Titanium-Pins (Figur 8A) die stärkste Artefaktbildung erfolgt. Hier ist ein Bereich durch Artefaktbildung nahe der Pinspitze komplett geschwärzt und für eine Analyse unbrauchbar. Auch bei Verwendung eines Zirkoniumoxid-Pins (Figur 8B) treten im Bereich des Pins starke Artefakte auf, diese sind aber fokussierter als bei Titanium und nicht so stark ausgeprägt. Ein deutlich besseres Ergebnis als bei der Verwendung von Titanium oder Zirkoniumoxid wird bei der Verwendung von SNI 750 erzielt. Dabei kommt es zwar im Bereich des Pins zur Artefaktbildung, diese Artefaktbildung ist aber wesentlich schwächer ausgeprägt. Das weitaus beste Ergebnis wurde bei der Verwendung von TECAMAX SRP-Pins (Figur 8D) erhalten. Die so erhaltenen Aufnahmen sind praktisch vollkommen frei von irgendwelchen Artefakten.

Die bei den Testaufnahmen hinsichtlich des Artefaktverhaltens gewonnen Resultate sind in Tabelle 2 zusammenfassend dargestellt:

**Tabelle 2**

| Material | Artefaktverhalten |
|---|---|
| Titanium | - |
| ZrO₂ | - |
| SNI 750 | 0 |
| TECAMAX SRP | ++ |

Die Verwendung von SNI 750-Pins liefert brauchbare Ergebnisse, hervorragende Ergebnisse werden bei Verwendung von TECAMAX SRP-Pins erhalten.

Die Ergebnisse hinsichtlich der Kriterien Biokompatibilität, Sterilisierbarkeit, mechanische Belastbarkeit und Artefaktverhalten, die für Pins aus unterschiedlichen Materialen erhalten wurden, die die Geometrie, die in Figur 5 gezeigt ist, aufweisen, sind in Tabelle 3 noch einmal insgesamt zusammengefasst:

**Tabelle 3**

| Material | 1 | 2 | 3 | 4 | Rangfolge |
|---|---|---|---|---|---|
| MACOR | + | + | - | n.a. | 7. |
| SNI750 | + | + | 0 | 0 | 2. |
| ZrO₂ | + | + | 0 | - | 3. |
| TECAMAX SRP | + | + | 0 | ++ | 1. |
| TECAPEEK classix | + | + | - | n.a. | 4. |
| TECAPEEK CF30 | 0 | + | - | n.a. | 5. |
| TECAPEEK GF 30 | 0 | + | - | n.a. | 6. |

| | | | | | |
|---|---|---|---|---|---|
| Legende 1 = Biokompatibilität, 2 = Sterilisierbarkeit, 3 = mechanische Beanspruchbarkeit, 4 = Artefaktverhalten | | | | | |

Dabei ist anzumerken, dass das Artefaktverhalten nur noch bei denjenigen Materialien untersucht wurde, bei denen auch die anderen drei Eignungskriterien eher auf eine insgesamt vorhandene Tauglichkeit des Materials als Befestigungsmittel zur Lagefixierung eines Körpers hingedeutet haben. Wurde eine bestimmte Eigenschaft für Befestigungsmittel bzw. Pins aus einem bestimmten Material nicht untersucht, so ist dies durch die Buchstabenkombination n. a. in der Tabelle 3 wiedergegeben.

Die besten Eigenschaften weist TECAMAX SRP auf. TECAMAX SRP erweist sich als biokompatibel und sterilisierfähig. Im Hinblick auf die mechanische Belastbarkeit wurden durchaus brauchbare Resultate erzielt. Hinsichtlich des Artefaktverhaltens weist TECAMAX SRP verglichen mit allen anderen Substanzen die besten Eigenschaften auf, die Aufnahmen sind praktisch vollständig artefaktfrei.

Das zweitbeste Gesamtresultat wurde für die getesteten Pins aus SNI 750 erhalten. Diese erfüllen die Anforderungen hinsichtlich Biokompatibilität und Sterilisierbarkeit und liefern hinsichtlich der Kriterien der mechanischen Belastbarkeit und Artefaktverhalten zumindest brauchbare Ergebnisse.

Auf Platz 3 der favorisierten Rangfolge liegen die Pins aus Zirkoniumoxid. Diese sind biokompatibel und sterilisierfähig und liefern auch hinsichtlich der mechanischen Beanspruchbarkeit zufrieden stellende Resultate. Negativ ist allerdings zu vermerken, dass bei Zirkoniumoxid störende Artefakte bei den CT-Scanns aufgetreten sind.

Als eher unvorteilhaft haben sich die Materialien TECAPEEK classix, TECAPEEK CF30 und TECAPEEK GF30 erwiesen, die allesamt Defizite bei der mechanischen Beanspruchbarkeit und meist nur gerade zufrieden stellende Resultate im Bereich der Biokompatibilität aufweisen. Als eher ungeeigneter Kandidat ist auch ein Befestigungsmittel aus MACOR anzusehen, dieses Material scheidet aufgrund der schlechten mechanischen Beanspruchbarkeit als Material für ein Befestigungsmittel zu Lagefixierung eines Körpers aus.

### 2. Versuchsreihe

Im Anschluss an die erste Testserie wurde eine zweite Testserie durchgeführt, um die beiden favorisierten Materialien TECAMAX SRP und SNI 750 weiter zu untersuchen und die Eigenschaften der daraus gefertigten Pins weiter zu verbessern. Insbesondere wurden Versuche unternommen, die mechanische Belastbarkeit der Pins zu verbessern. Zu diesem Zweck wurde die Geometrie der Pins verändert, und es wurden einige Prototypen hergestellt.

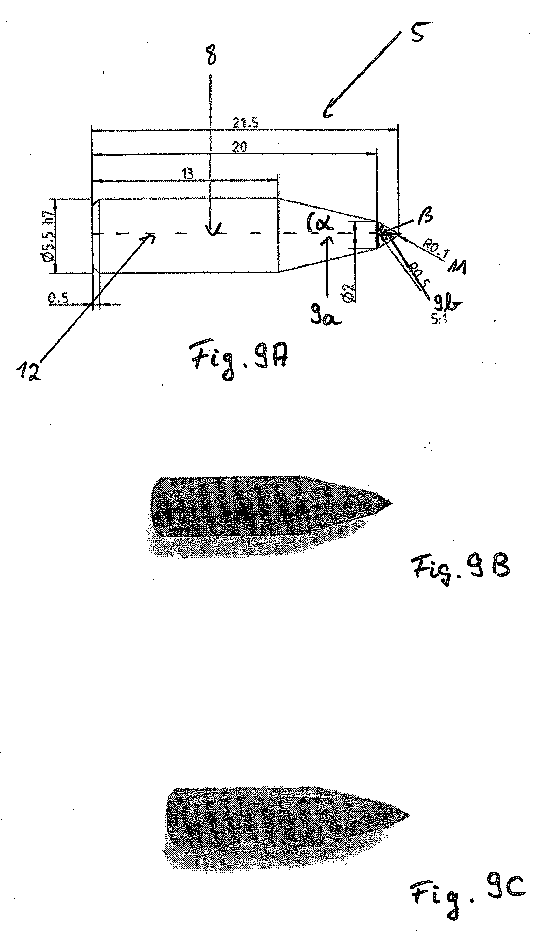

In Figur 9A ist eine schematische Zeichnung eines Pins mit veränderter Geometrie dargestellt, Figuren 9B und 9C zeigen Abbildungen eines TECAMAX SRP-Pins und eines SNI 750-Pin mit der in Figur 9A dargestellten Geometrie.

Der in Figur 9A dargestellte Pin 5 weist einen Halteabschnitt und einen Eindringabschnitt 9 auf, wobei der Eindringabschnitt eine Spitze 11 aufweist und wobei der gesamte Eindringabschnitt 9 nicht die Form eines Kegels hat. Stattdessen lässt sich der Eindringbereich 9 in zwei Bereiche 9a und 9b unterteilen. Bei 9a handelt es sich um einen Bereich des Eindringabschnittes, der auf der einen Seite an den Halteabschnitt angrenzt. Er ist kegelstumpfförmig ausgebildet. An der Schnittfläche des Kegelstumpfes, die dem Halteabschnitt 8 gegenüberliegt, grenzt der zweite Bereich 9b an. Dieser ist kegelförmig ausgebildet und weist eine Spitze 11 auf. Der gesamte Pin weist eine Längsachse 12 auf, um die herum der Pin 5 achsenrotationssymmetrisch ausgebildet ist. Die äußere Hülle des kegelstumpfförmigen Bereichs 9a weist einen Winkel α zur Rotationsachse 12 auf. Der Kegelmantel des kegelförmigen Bereiches 9b weist einen Winkel β zur Rotationsachse 12 auf. Der Winkel α ist spitzer ausgebildet als der Winkel β. Der Winkel α liegt bevorzugt im Bereich von 20° bis 30°, bevorzugt im Bereich 22° bis 26°, und höchst bevorzugt bei 24°; dabei gilt für jeden vorstehend exakt bezeichneten Wert eine Toleranz von ± 0,2°. Der Winkel β liegt bevorzugt im Bereich von 30° bis 37°, bevorzugt im Bereich von 32° bis 35°, und höchst bevorzugt bei 33,5°; dabei gilt für jeden vorstehend exakt bezeichneten Wert eine Toleranz von ±0,2°. Dies hat zu Folge, dass der Bereich 9b, der direkt zur Lagefixierung in einen Körper eindringt, weniger spitz ausgebildet ist als der Bereich 9a, der außerhalb des Körpers verbleibt. Wie in der Versuchen zur Messung der Eindringtiefe der Pins festgestellt wurde, dringen die Pins um höchstens 1,5 mm in einen Körper ein. Diese Mindestlänge von 1,5 mm weist der kegelförmige Bereich 9b in Richtung der Achse 12 auch mindestens auf.

Grundsätzlich wurde gefunden, dass weniger Artefakte verursacht wurden, je weniger spitz der Artefakt-bildende Gegenstand ausgebildet ist. Kleine Abweichungen in der Geometrie der Pins, d. h. bei den verwendeten Winkeln, führen aber nicht zu wesentlichen Unterschieden in der Artefaktausprägung.

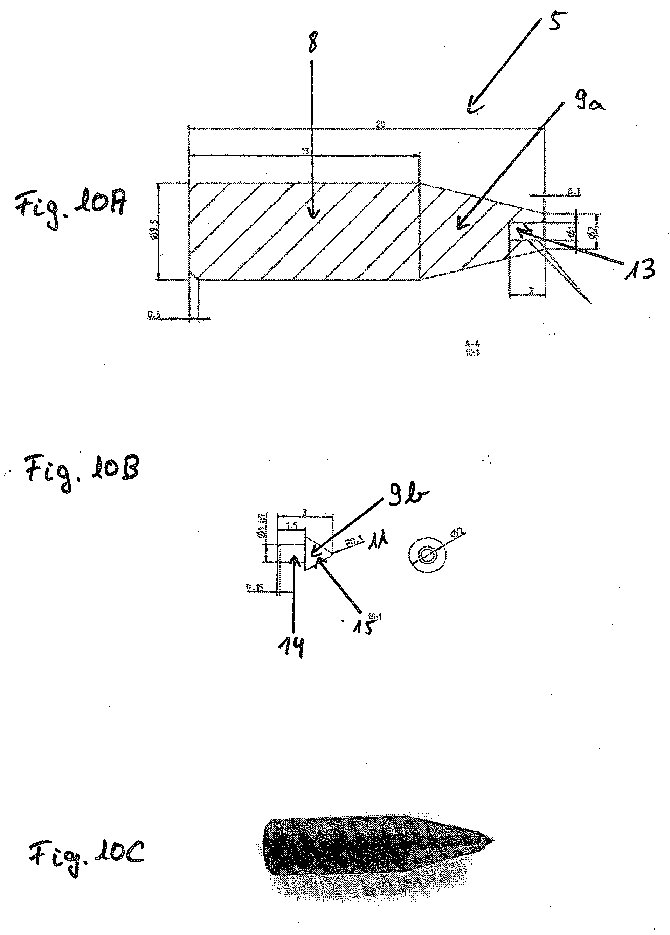

Während der in Figur 9A gezeigte Pin 5 insgesamt einstückig ausgebildet ist und deshalb aus einem einzigen Material hergestellt ist, zeigen die Figuren 10A und 10B in schematischer Schnittdarstellung einen Pin, der zweistückig ausgebildet ist. Das erste Stück setzt sich aus einem Halteabschnitt 8 und einem ersten Bereich des Eindringabschnitts 9a zusammen. Der Bereich 9a des Eindringabschnittes 9 weist einen Hohlraum 13 auf Dieser ist im dargestellten Beispiel zylinderförmig ausgebildet. In diesen Hohlraum 13 hinein kann nun der zweite Bereich 9b des Eindringabschnittes 9 eingefügt werden. Dies kann z. B. durch Vorsehen eines Pressfittes durch Kleben oder Umspritzen geschehen. Der zweite Bereich 9b des Eindringabschnitts 9 ist bevorzugt einstückig ausgebildet und besteht aus ein und demselben Material. Alternativ ist es auch möglich, diesen Bereich 9b zweistückig auszubilden. Der Bereich 9b setzt sich aus einem Kopfstück 15, das kegelförmig ausgebildet ist und eine Spitze 11 aufweist, und aus einem Fortsatz 14, der im vorliegenden Beispiel zylinderförmig ausgebildet ist, zusammen. Der Fortsatz 14 wird in den Hohlraum 13 eingefügt. In zusammengesetztem Zustand entsprechen die geometrischen Verhältnisse des in Figuren 10A und 10B dargestellten so genannten "Hybrid-Pins" den Verhältnissen, die in Figur 9A dargestellt sind. Das bedeutet insbesondere, dass der Eindringabschnitt 9 Bereiche 9a und 9b aufweist, deren Umhüllung bezüglich der Achse 12 verschiedene Winkel α und β aufweist, wobei wiederum der Winkel α zwischen den Kegelstumpf und der Achse und der Winkel β zwischen den Kegel und der Achse gebildet wird, und wobei der Winkel α spitzer ist als der Winkel β.

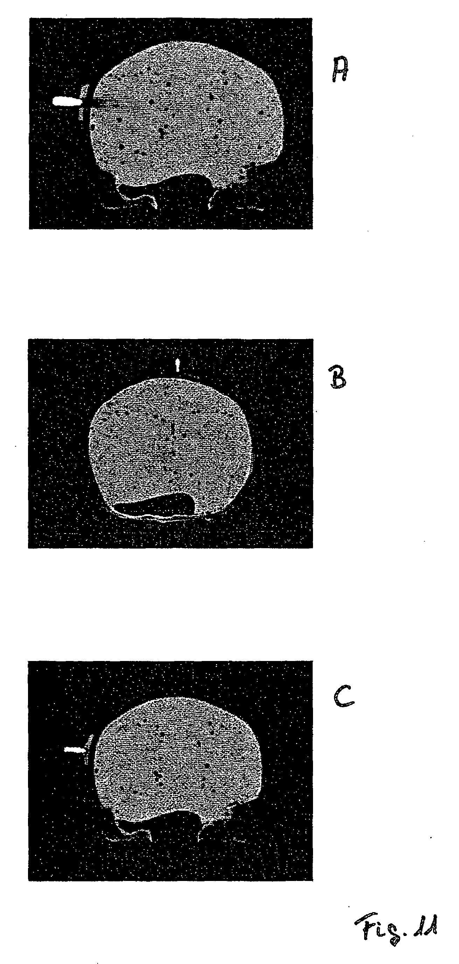

Vorteilhaft bei dem in den Figuren 10A und 10B dargestellten Hybrid-Pin ist, dass der Halteabschnitt und der erste Bereich des Eindringabschnittes 9a aus einem ersten Material und der zweite Bereich des Eindringabschnitts 9b aus einem anderen Material hergestellt werden können. Dabei ist es möglich, den zweiten Bereich 9b des Eindringabschnitts 9 z. B. aus einer Keramik zu fertigen. Das übrige Befestigungsmittel mit dem Halteabschnitt 8 und dem ersten Bereich 9a des Eindringabschnitts 9 könnte z. B. aus TECAMAX SRP gefertigt werden. Dies wurde beispielhaft auch für einen untersuchten Hybrid-Pin verwirklicht, der in Figur 10C dargestellt ist. Pins aus den Materialien TECAMAX SRP und SNI 750, die einstückig ausgebildet waren und die in Figur 9A gezeigte Geometrie aufwiesen wurden hinsichtlich ihrem Artefaktverhalten getestet. Des Weiteren wurde ein Hybrid-Pin getestet, dessen zweiter Bereich 9b des Eindringabschnitts samt Spitze 11 aus SNI 750 bestand, während das übrige Befestigungsmittel mit Halteabschnitt 8 und erstem Bereich 9a des Eindringabschnitts 9 aus TECAMAX SRP gefertigt wurde. Für jeden dieser Pins wurde eine Serie von CT-Scanns durchgeführt, exemplarisch sind einige Aufnahmen in Figur 11 dargestellt. Figur 11A zeigt einen SNI 750-Pin mit verbesserter Geometrie, Figur 11B zeigt einen Hybrid-Pin mit verbesserter Geometrie (Kombination TECAMAX SRP / SNI 750), und Figur 11C zeigt einen TECAMAX SRP-Pin mit verbesserter Geometrie. Nur die Aufnahme, die in Figur 11A gezeigt ist, zeigt noch leichte Artefakte auf. Dennoch handelt es sich um eine insgesamt recht brauchbare Aufnahme. Die Aufnahmen in Figur 11B und 11C sind praktische artefaktfrei und von sehr guter Qualität. Vergleicht man Figur 11A mit Figur 11C (SNI 750 mit TECAMAX SRP), so erkennt man, dass durch die Fertigung eines Hybrid-Pins, bei dem nur ein kleiner Bereich, der die Spitze enthält, aus SNI 750 gefertigt ist, der größere Bereich jedoch aus TECAMAX SRP gefertigt ist, man die positiven Artefakteigenschaften von TECAMAX SRP nahezu vollständig ausschöpfen kann. Es ist also für die guten Eigenschaften hinsichtlich Artefakt-Bildung nicht notwendig, dass der verwendete Pin gänzlich aus einem besonders gut Artefakte unterdrückenden Material wie TECAMAX SRP gefertigt ist.

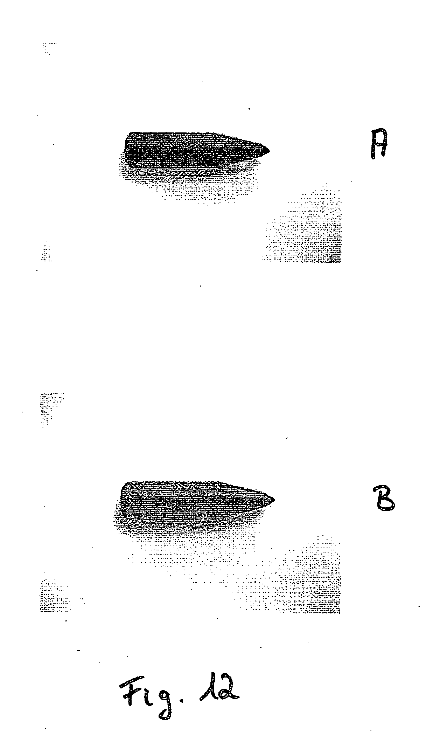

Die für die Aufnahmen, die in Figur 11 dargestellt sind, verwendeten Pins wurden nun mechanischen Belastungstest unterzogen. Diese wurden wie bei der ersten Versuchsreiche auch an Leichen in der Pathologie unter Mitarbeit von Neurochirurgen durchgeführt. Dabei zeigte sich, dass die Pins aus TECAMAX SRP und SNI 750 verglichen mit den Pins der ersten Versuchsreihe, die einen nicht optimierte Geometrie aufwiesen, bessere Testergebnisse erzielten. In Figur 12A ist ein TECAMAX SRP-Pin dargestellt, in Figur 12B ist ein SNI 750-Pin jeweils nach dem Test dargestellt; bei den dargestellten Pins handelt es sich jeweils um den am stärksten deformierten bzw. beschädigten Pin. In beiden Fällen ist praktisch keine Deformation oder ein Defekt zu erkennen. Lediglich bei der Verwendung des Hybrid-Pins traten hinsichtlich der mechanischen Beanspruchbarkeit ab bestimmten Beanspruchungen Probleme auf.

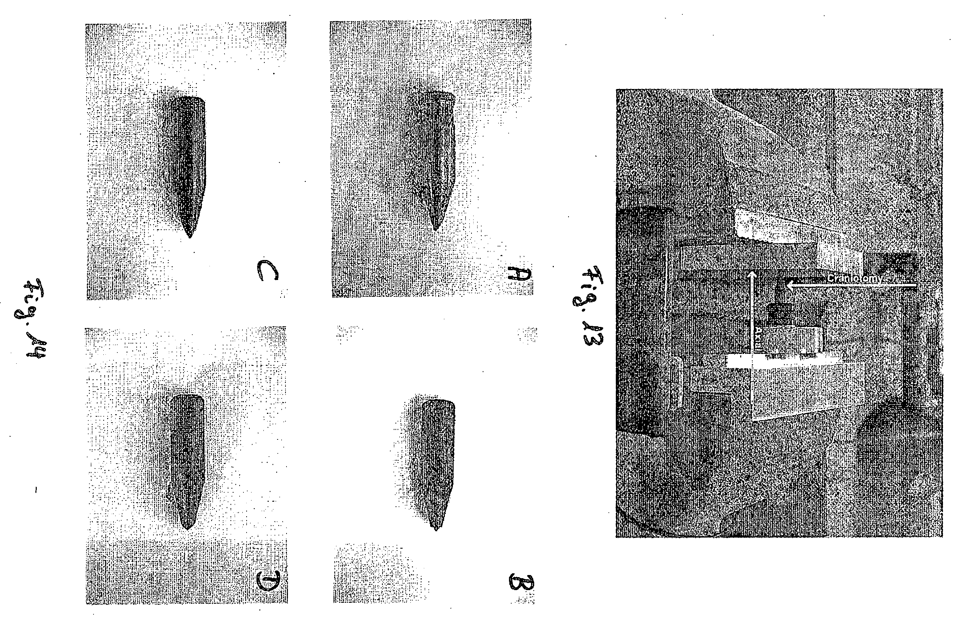

Um die mechanische Beanspruchbarkeit der Pins noch genauer zu untersuchen, wurden die Pins in eine Versuchsanordnung eingespannt, die in Figur 13 dargestellt ist. In einer Zwinge wurden sie mit einer axialen Kraft beaufschlagt, die durch einen Sensor jeweils gemessen wurde. Die maximale Krafteinwirkung betrug 2.000 N, Dies würde einem Sicherheitsfaktor von mehr als 5,5 verglichen mit dem bei einer Kraniotomie tatsächlich auftretenden Kräften entsprechen. Während des Versuches wurden die Pins gegen eine Aluminium-Platte gepresst, um den Knochen eines Patienten zu simulieren. Dabei ist zu berücksichtigen, dass die Aluminium-Platte wesentlich härter ist als ein menschlicher Knochen, weshalb die erhaltenen Ergebnisse eine sehr konservative Abschätzung der maximal auszuhaltenden mechanischen Beanspruchbarkeit darstellen.

Mit der in Figur 13 dargestellten Versuchsanordnung wurden Pins aus den Materialien TECAMAX SRP, SNI 750 und ein Hybrid-Pin (Kombination TECAMAX SRP / SNI 750) untersucht. Für Vergleichszwecke wurde ein Titanium-Pin untersucht. Nach Durchführung der Tests wurden die Pins visuell untersucht, sie sind in Figur 14A bis 14D abgebildet. Der in Figur 14A dargestellte Titanium-Pin hielt die axiale Beanspruchung von 2.000 N aus, er verformte sich lediglich im Bereich der Spitze leicht. Der in Figur 14C gezeigte SNI 750-Pin hielt ebenfalls der axialen Belastung von 2.000 N stand. Gleiches gilt für den TECAMAX SRP-Pin, der in Figur 14D dargestellt ist. Lediglich bei dem Hybrid-Pin (Figur 14B) kam es bei einer Einwirkung von 400 N zum Bruch. Dies lässt darauf schließen, dass die Hybrid-Pins in der derzeitigen Ausführungsform bei der Verwendung als Befestigungsmittel zur Lagefixierung eines Körpers gewissen Einschränkungen unterworfen sind; dabei ist aber zu berücksichtigen, dass die unterschiedlichen Pins bei Verwendung einer Kopfklemme mit Drei-Punkt-Fixierung stark unterschiedlichen Kräften ausgesetzt sind. Es dürfte lediglich bei der Verwendung von Hybrid-Pins für Pin 1, der den stärksten Belastungen ausgesetzt ist, mit Problemen zu rechnen sein.

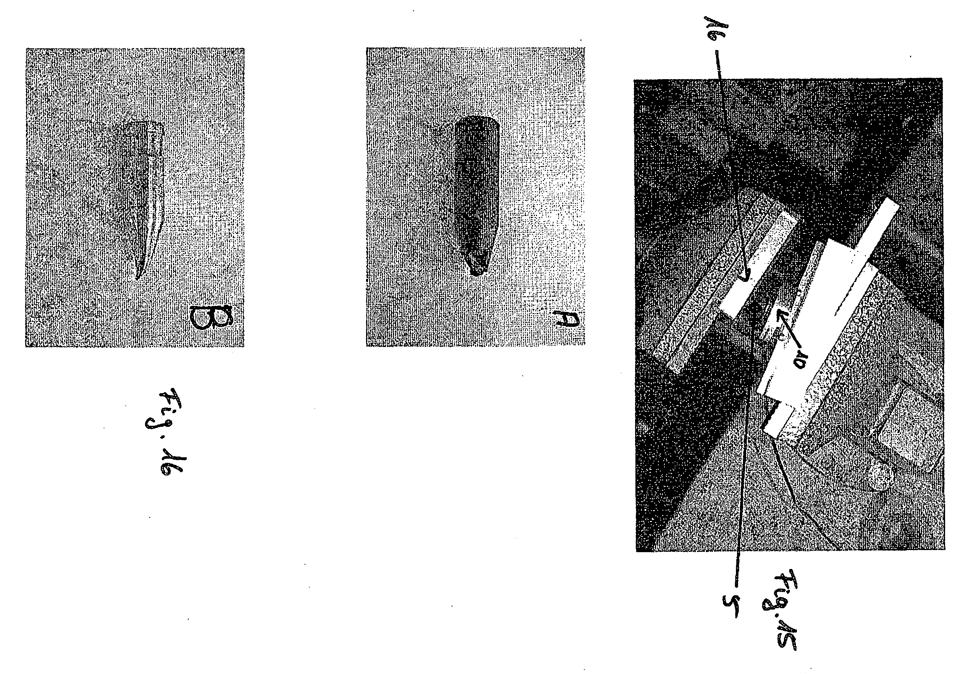

Schließlich wurden die Pins aus TECAMAX SRP, SNI 750 und ein Vergleichspin aus Stahl einem Experiment unterworfen, wo zusätzlich zu den hohen Kräften in axialer Richtung auch Biegemomente einwirkten. Ein Versuchsaufbau ist in Figur 15 abgebildet. Zu erkennen ist, dass ein Pin 5 schräg auf eine Fläche 16 gepresst wird. Die einwirkende Kraft wurde mit einem Sensor 10 gemessen. Der Pin aus TECAMAX SRP (Figur 16A) brach bei einer Beanspruchung von 1.850 N. Dieser Wert liegt weit über den tatsächlich bei einer Craniotomie auftretenden Kräften. Der in Figur 16B gezeigte Stahl-Pin hielt auch den auftretenden Scherkräften und der Gesamtbeanspruchung von 2.000 N stand, allerdings verformte sich auch bei ihm bereits die Spitze.

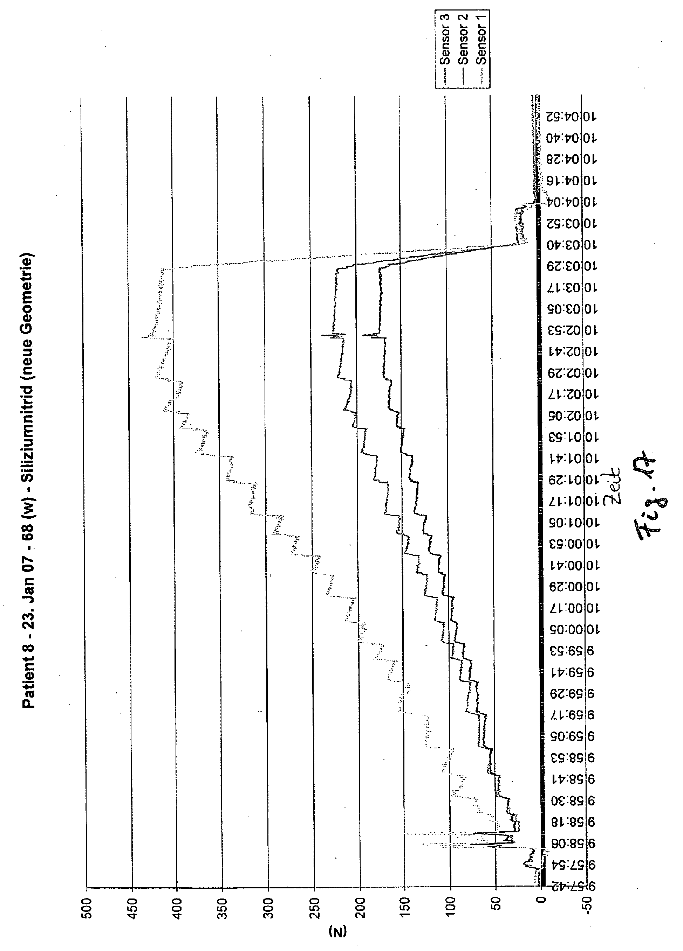

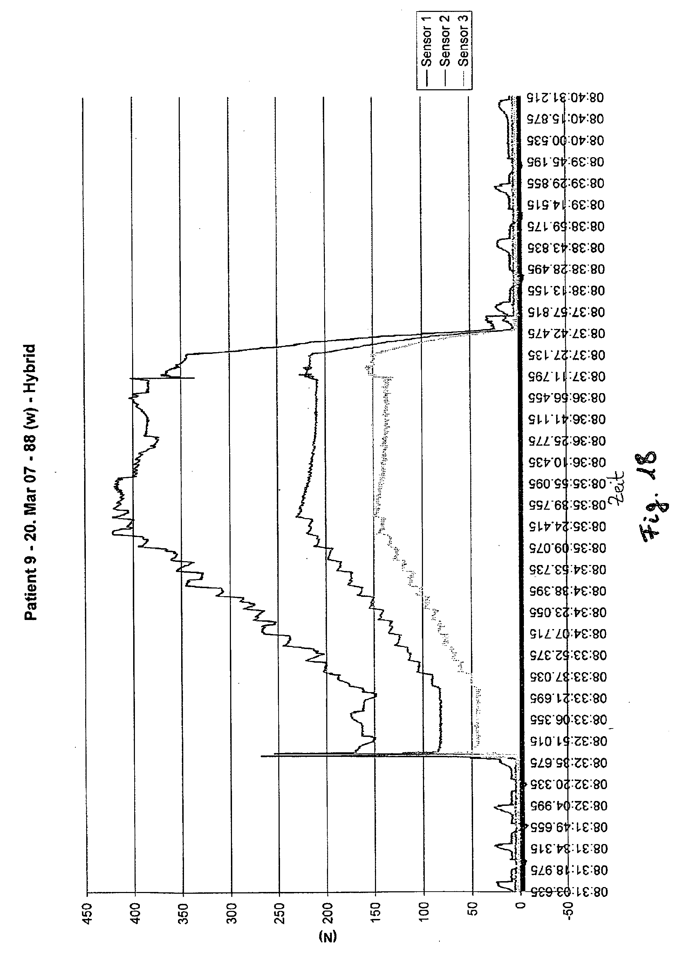

In den Figuren 17 und 18 sind Messungen der einwirkenden Axialkräfte über der Zeit aufgetragen, wie sie bei den Versuchen im Pathologischen Institut mit Pins der verbesserten Geometrie gemessen wurden. In Figur 17 wurde ein Pin aus Siliziumnitrid mit der verbesserten Geometrie verwendet, in Figur 18 wurde ein Hybrid-Pin (TECAMAX SRP /SNI 750) verwendet. Im Prinzip unterscheiden sich die auftretenden Kräfte nicht von denen, die bei Verwendung eines Pins mit der ursprünglichen Geometrie (vergleiche Figur 5) erhalten wurden.

In Tabelle 4 sind die Ergebnisse der zweiten Versuchsreihe noch einmal in einer Übersicht dargestellt:

**Tabelle 4**

| Material | 1. | 2. | 3. | 4. | Rangfolge |
|---|---|---|---|---|---|
| SNI 750 | ++ | ++ | ++ | 0 | 2. |
| Hybrid-Pin | ++ | + | - | + | 3. |
| TECAMAX SRP | ++ | ++ | + | ++ | 1. |

| | | | | | |
|---|---|---|---|---|---|
| Legende 1 = Biokompatibilität 2 = SteriHsierungsfahigkeit 3 = mechanische Beanspruchbarkeit 4 = Artefaktverhalten | | | | | |

Die besten Materialeigenschaften weist ein Pin aus TECAMAX SRP auf, dicht gefolgt von einem Pin aus SNI 750. Verglichen mit der ersten Versuchsreihe sind bei der zweiten Versuchsreihe die Eigenschaften betreffend die mechanische Beanspruchbarkeit bei TECAMX SRP und SNI 750 noch einmal verbessert. Die beste mechanische Beanspruchbarkeit weist ein Pin aus SNI 750 auf, dicht gefolgt von TECAMAX SRP. Hinsichtlich der mechanischen Beanspruchbarkeit schneidet der Hybrid-Pin (TECAMAX SRP / SNI 750) etwas schlechter ab, wie bereits ausgeführt ist der Hybrid-Pin zwar im Prinzip einsatzbereit, jedoch mit Einschränkungen. Hinsichtlich des Artefaktverhaltens liefert TECAMAX SRP hervorragende Ergebnisse, der Hybrid-Pin (TECAMAX SRP / SNI 750) liefert ebenfalls Ergebnisse hoher Qualität, wenn auch nicht ganz so hoher Qualität wie bei der Verwendung der TECAMAX SRP-Pins. Ein Pin aus SNI 750 mit der verbesserten Geometrie der zweiten Versuchsreihe überzeugt durch gute Werte bei Biokompatibilität, Sterilisierbarkeit und mechanischer Beanspruchbarkeit. Bei der mechanischen Beanspruchbarkeit erzielt ein Pin aus SNI 750 Bestwerte. In diesem Fall sind allerdings leichte Abstriche hinzunehmen hinsichtlich des beobachteten Artefaktverhaltens.

Zusammengefasst liefern alle in Tabelle 4 aufgeführten Materialien für Befestigungsmittel bzw. Pins zur Lagefixierung eines Körpers gegenüber dem Stand der Technik deutlich verbesserte Ergebnisse. Diese wurden zum einen aufgrund der geeigneten Materialauswahl, und zum anderen aufgrund der verbesserten und angepassten Geometrie erzielt.

### Zusammensetzung MACOR

46 % Siliziumoxid
17 % Magnesiumoxid
16 % Aluminiumoxid
10% Kaliumoxid
7% Boroxid
4 % Fluor

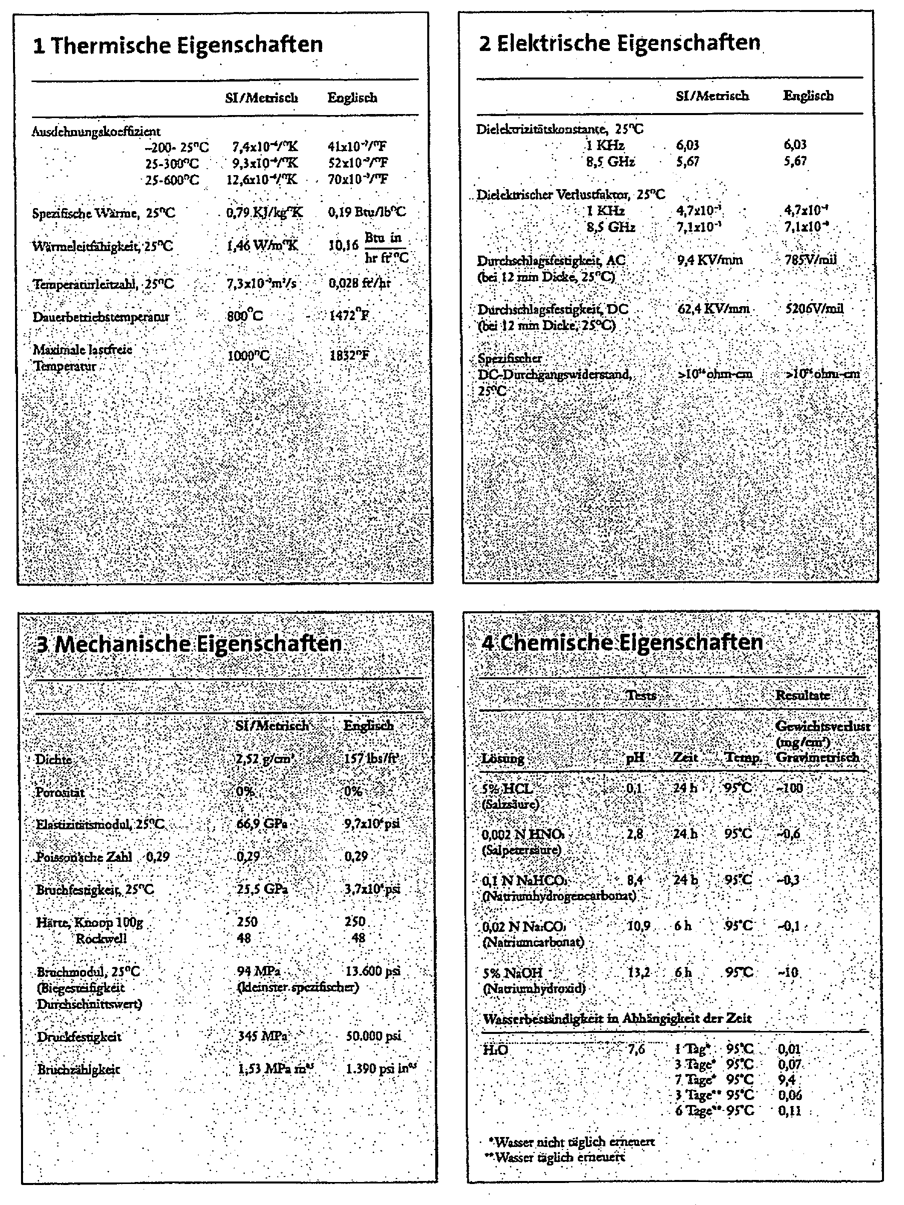

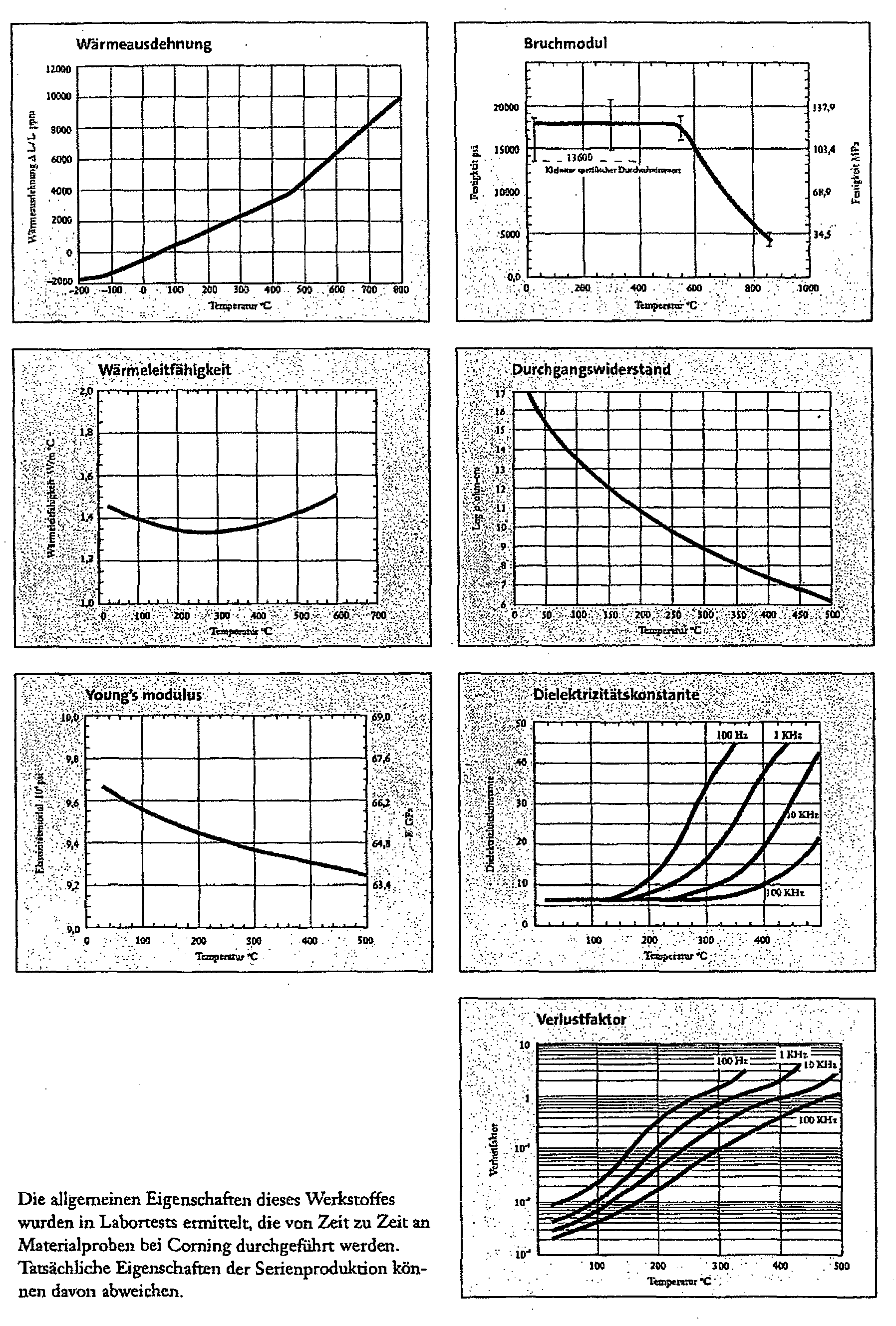

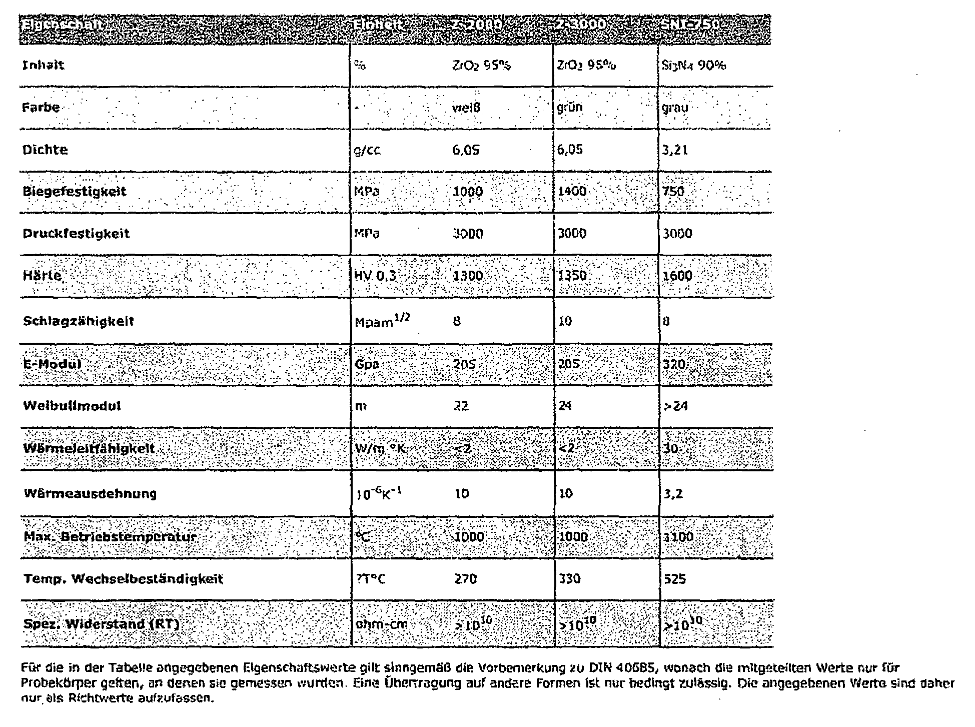

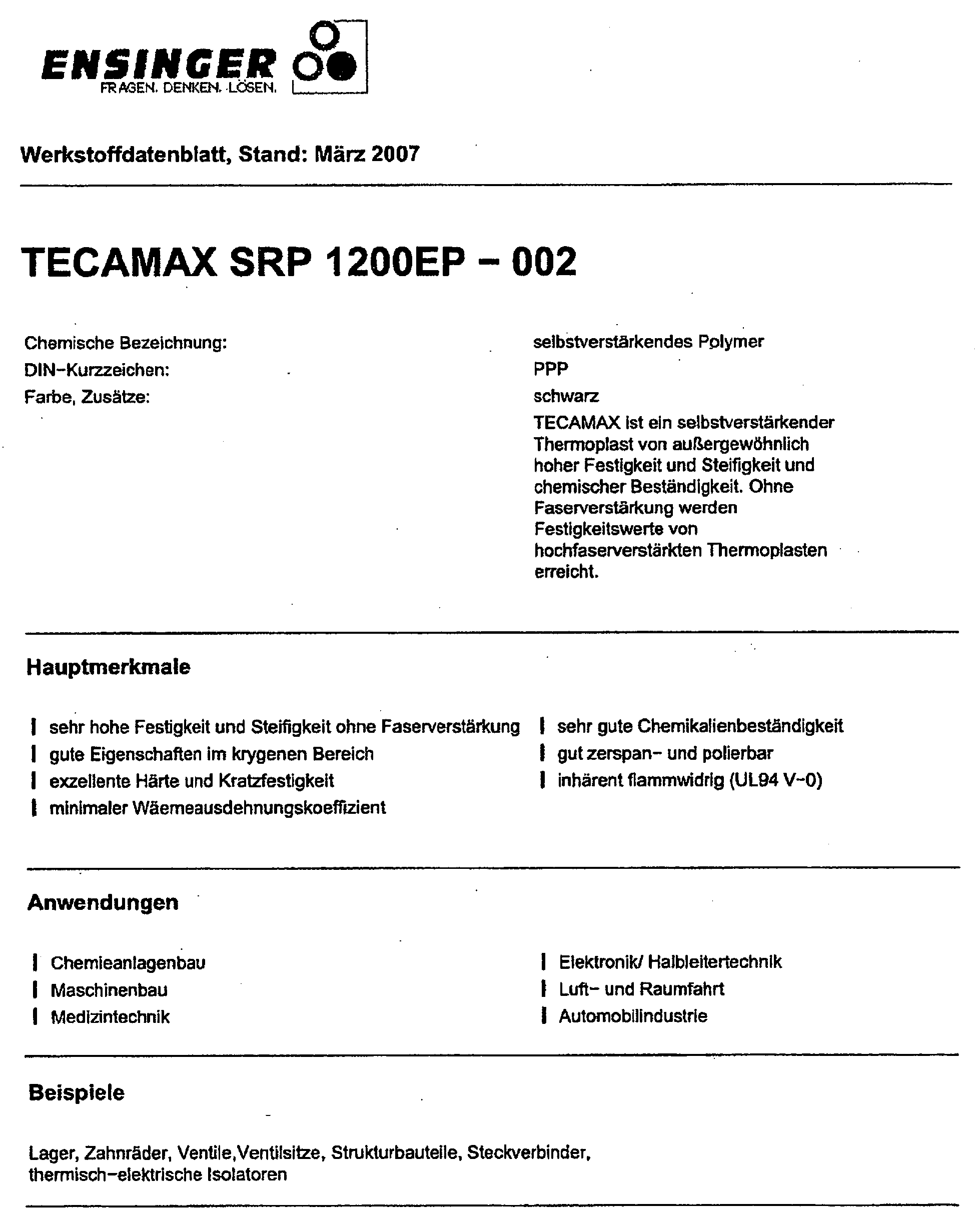

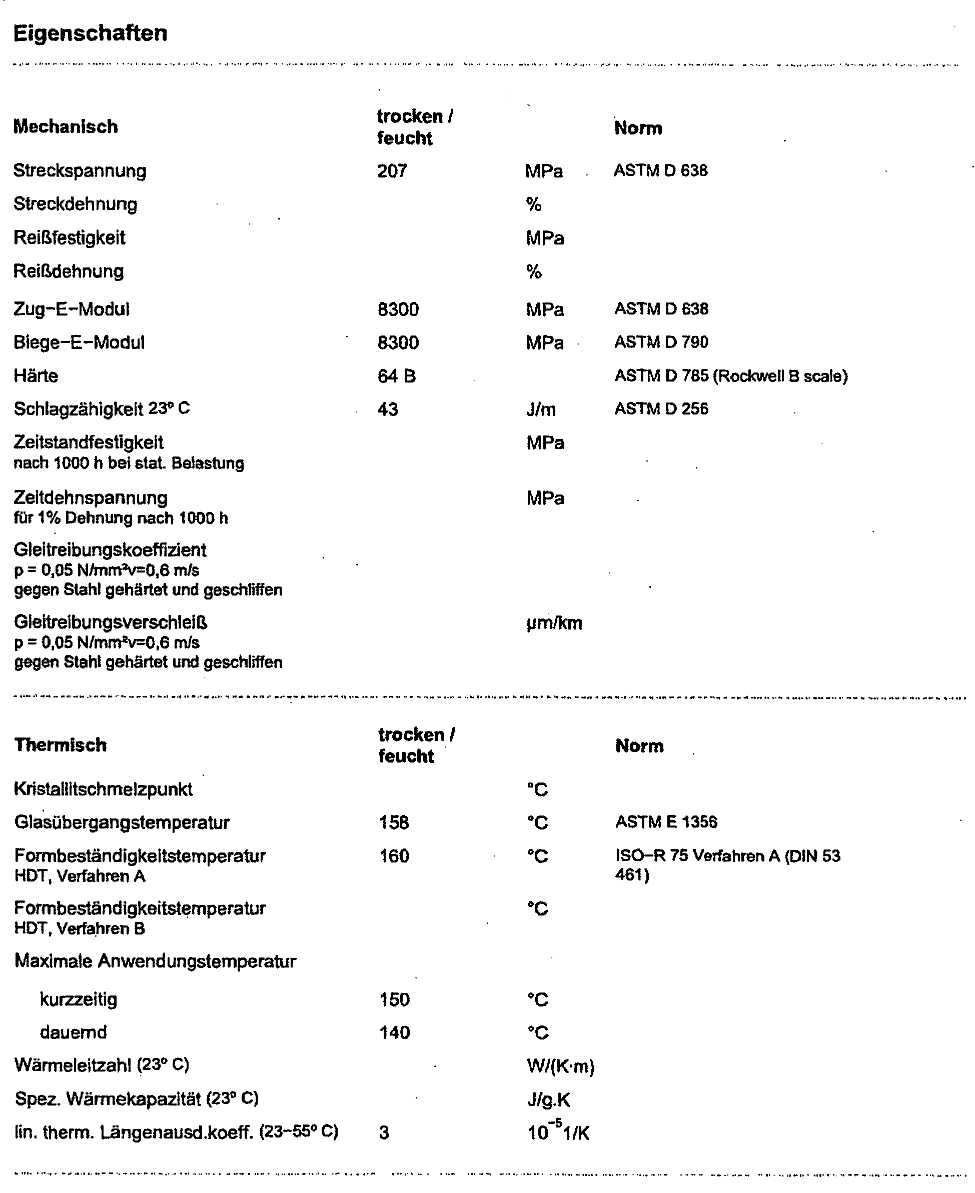

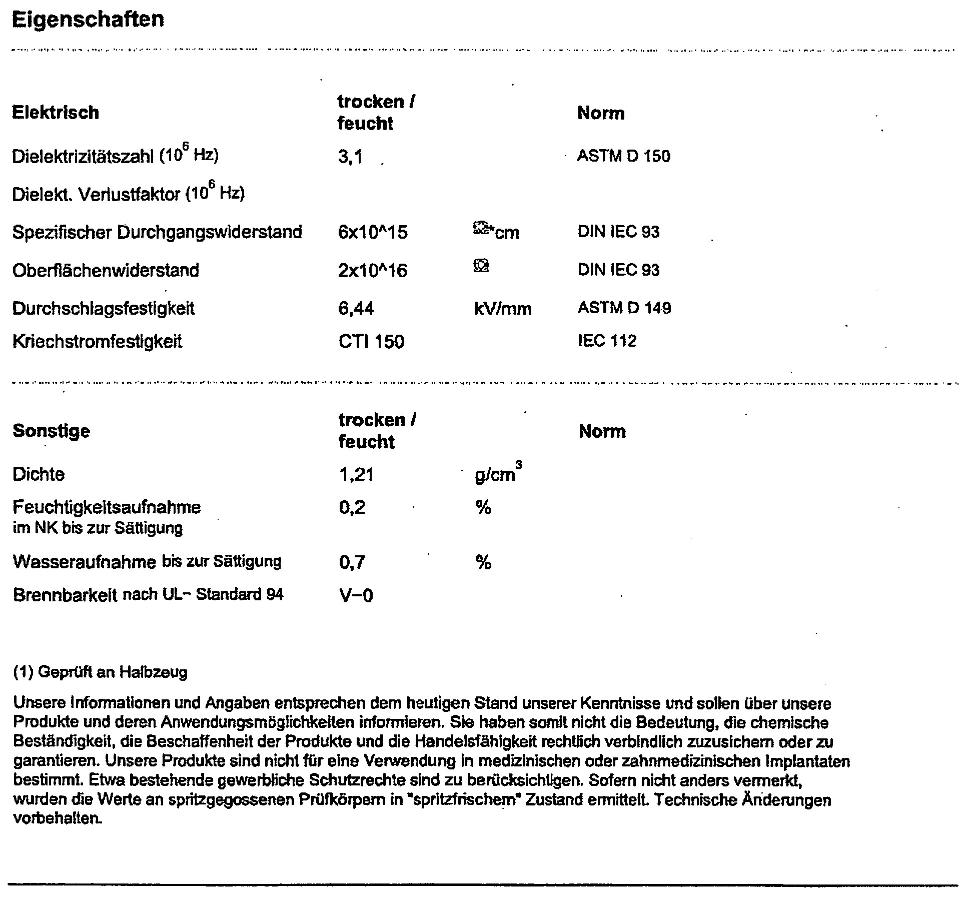

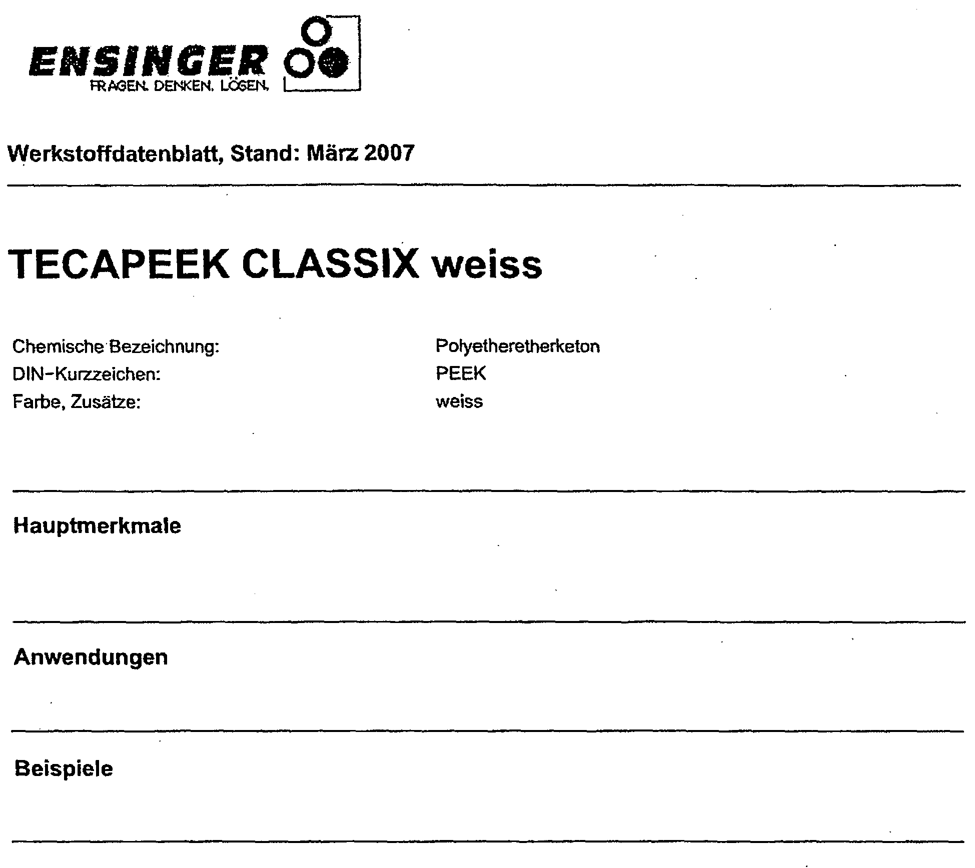

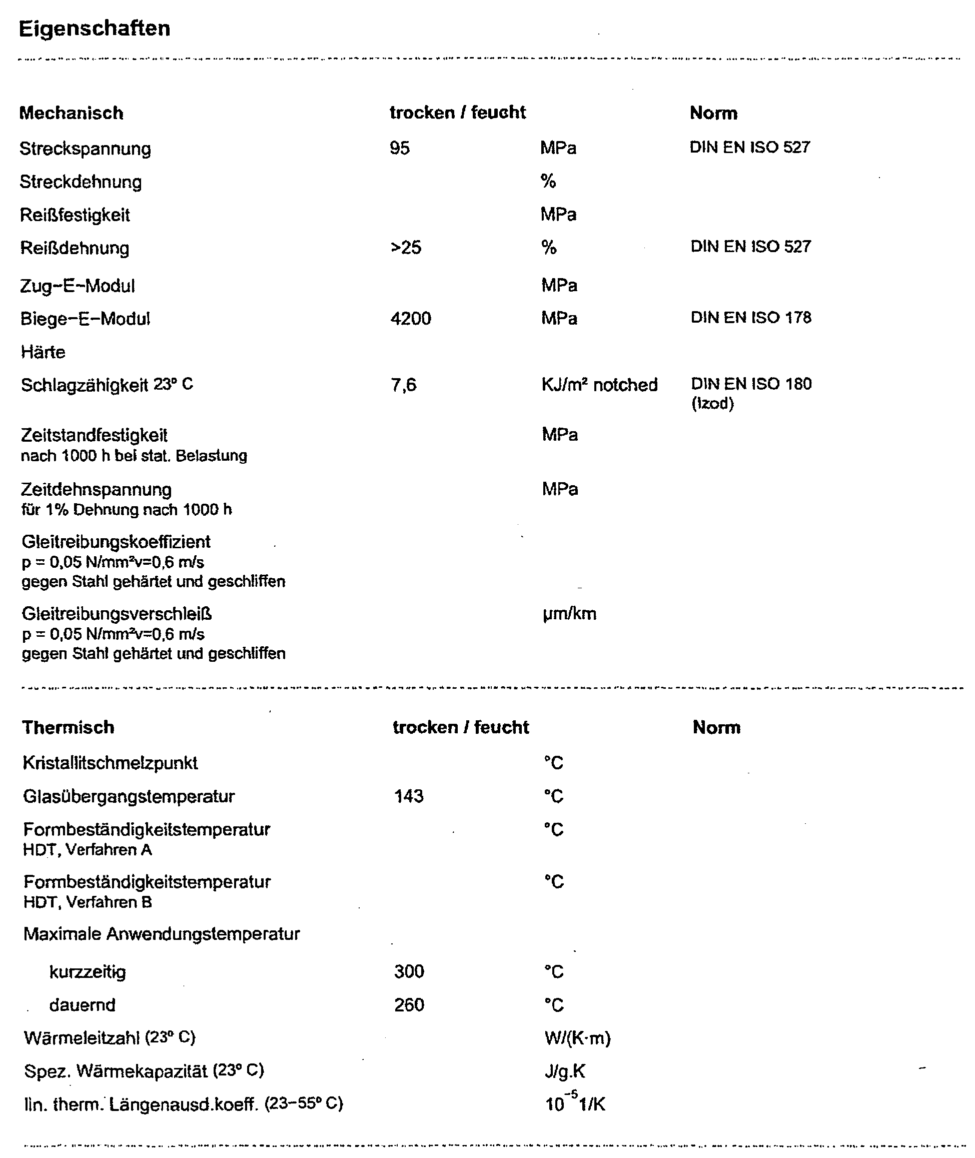

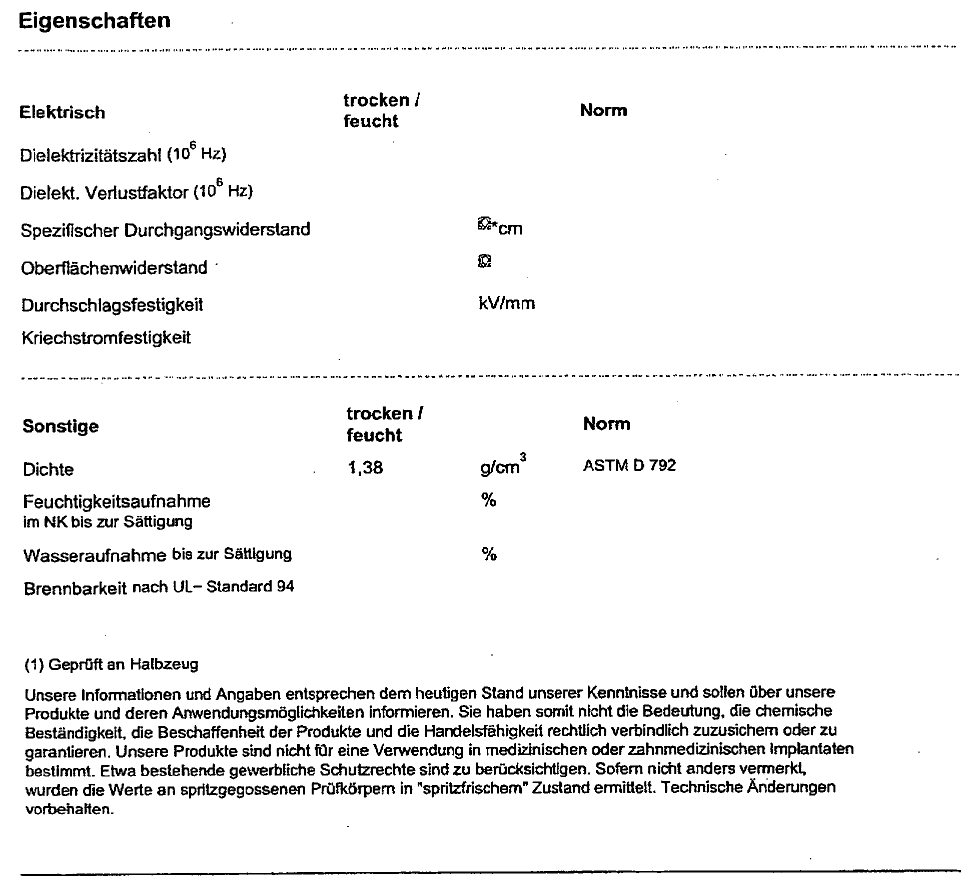

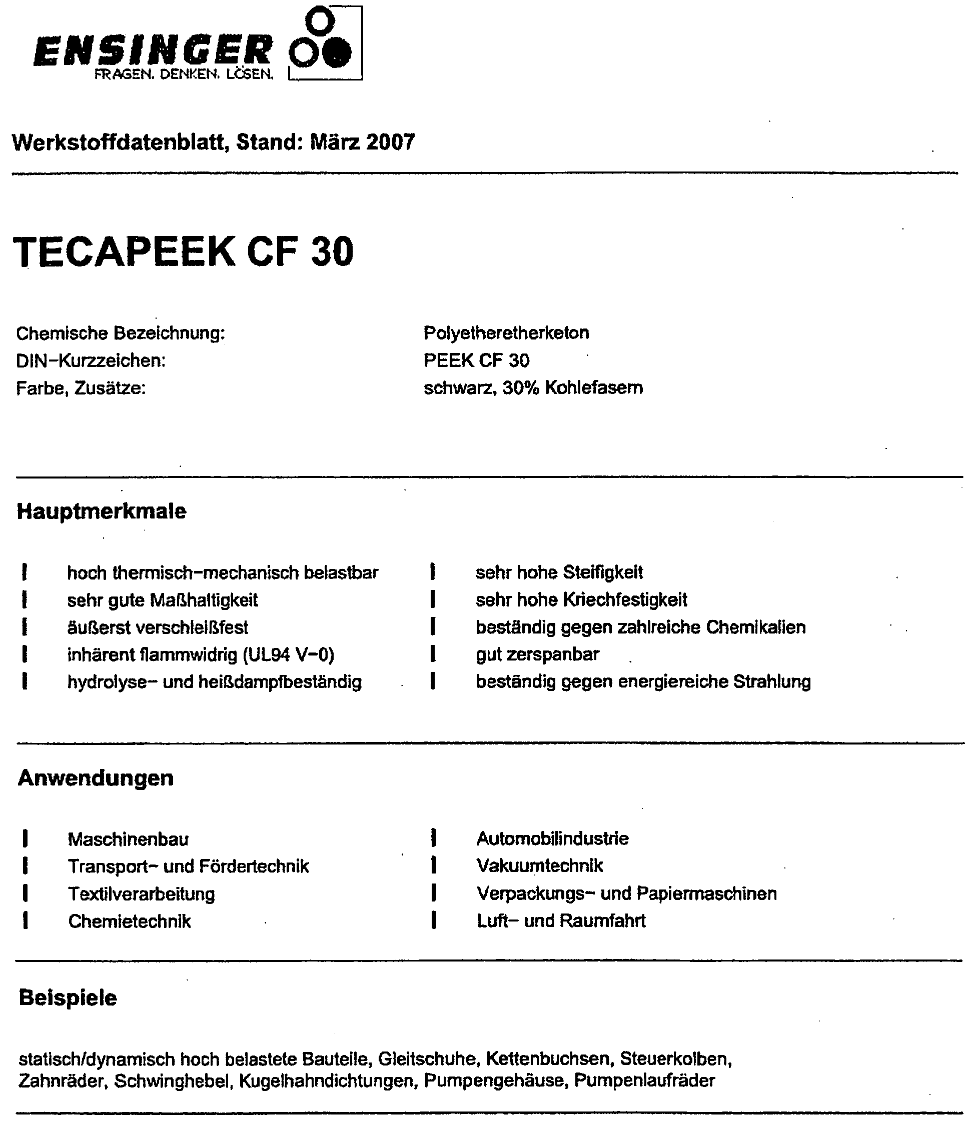

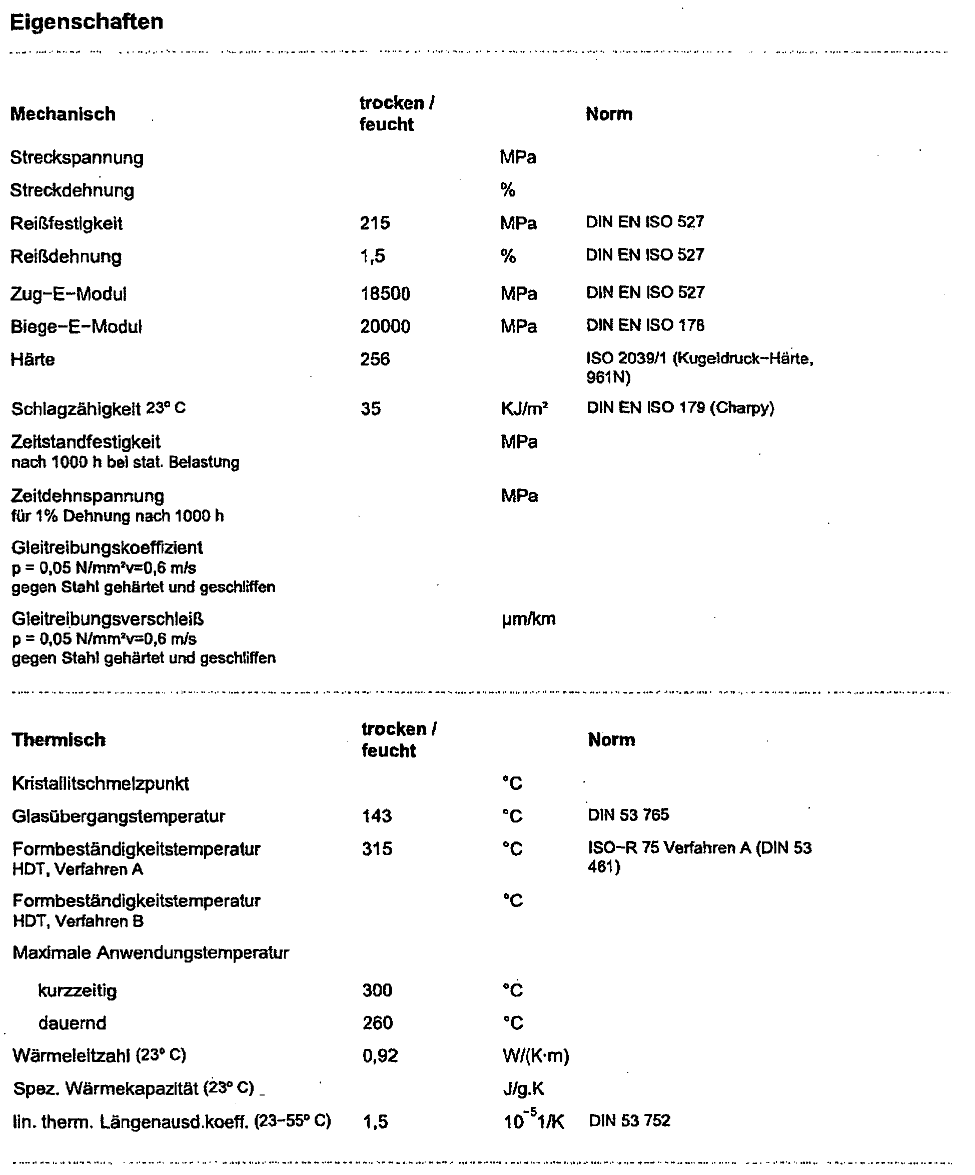

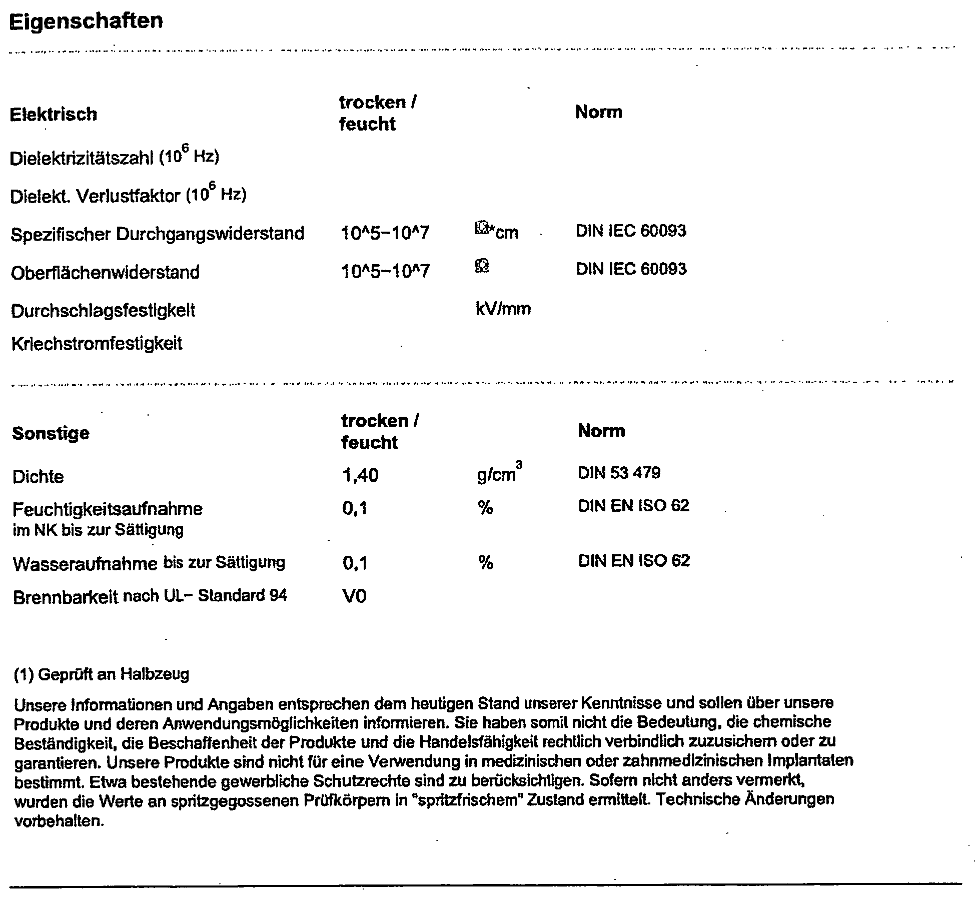

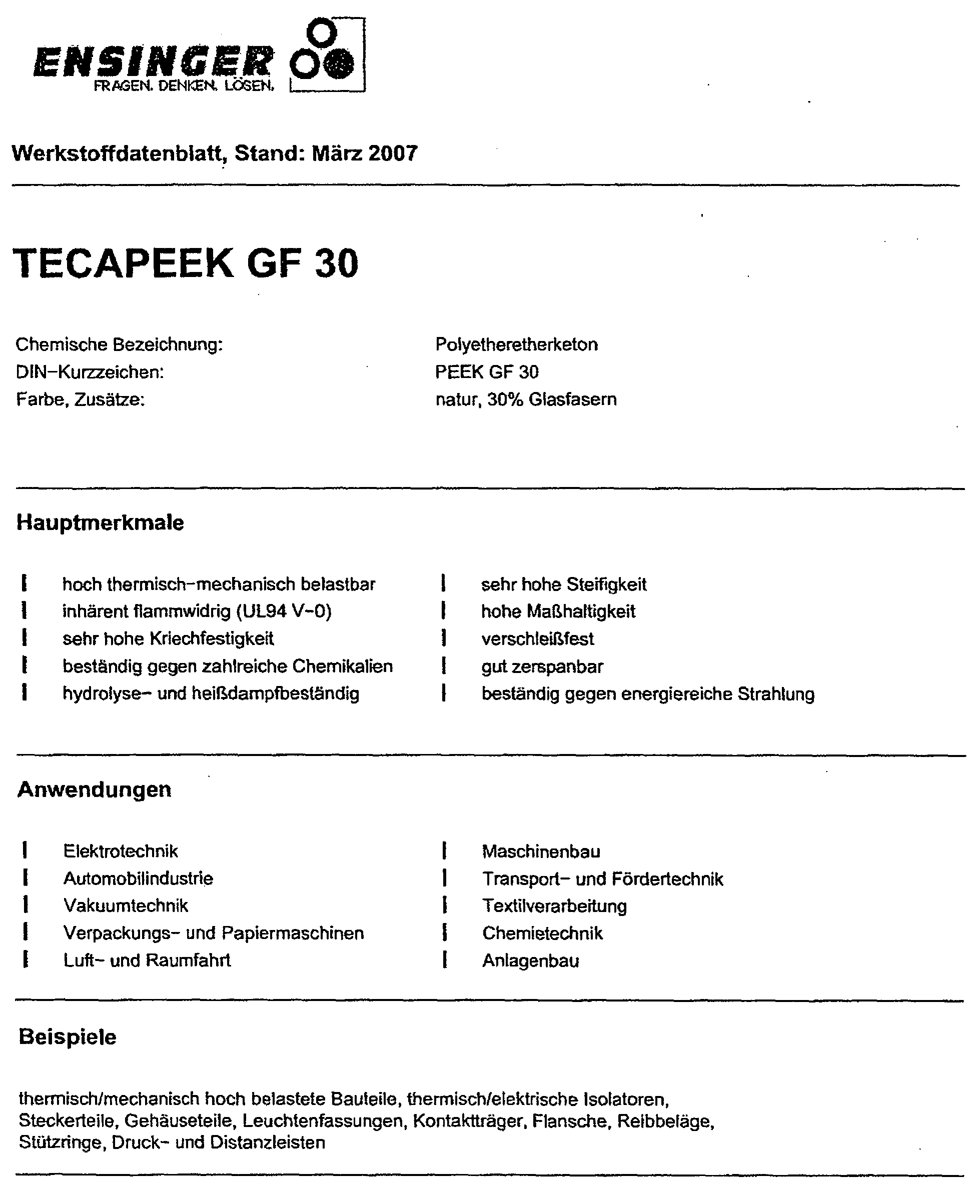

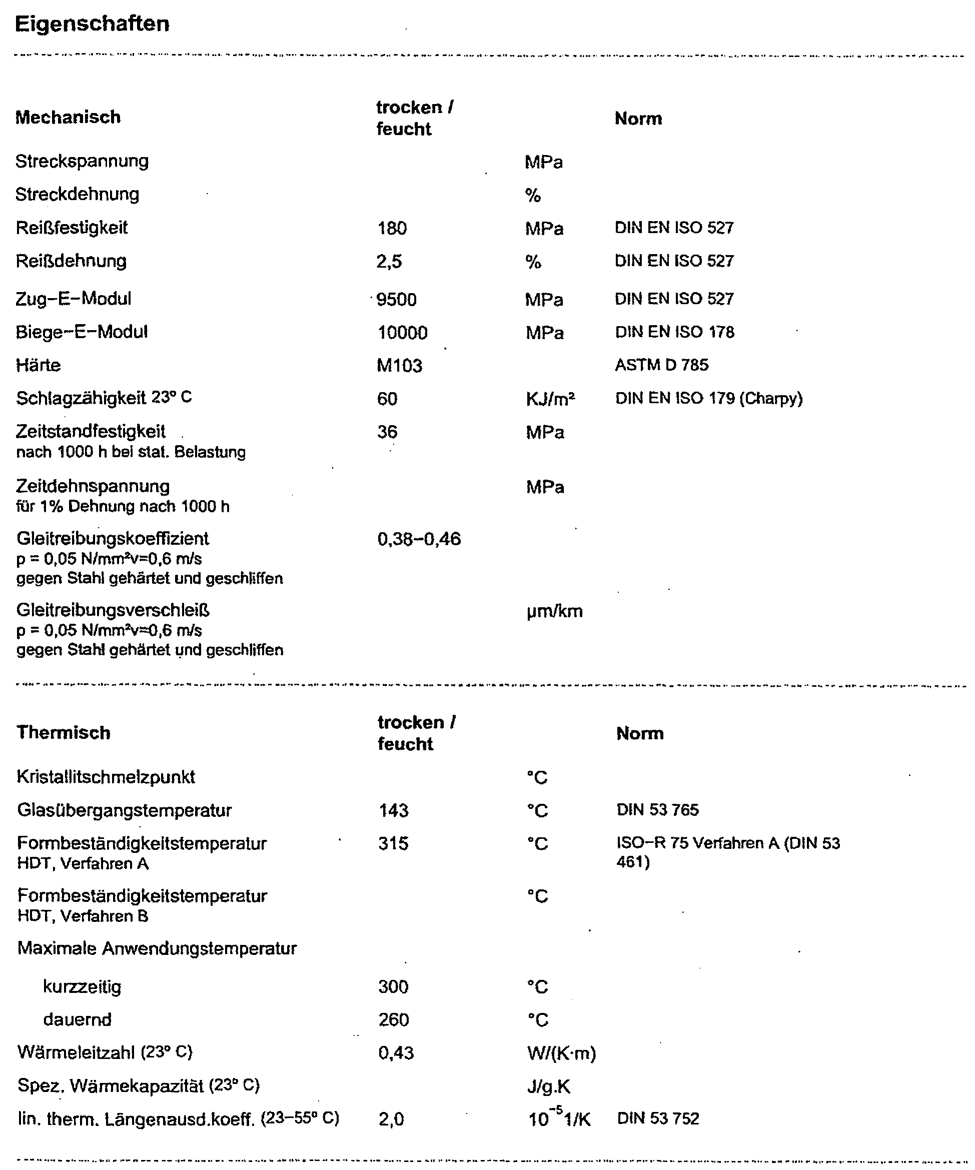

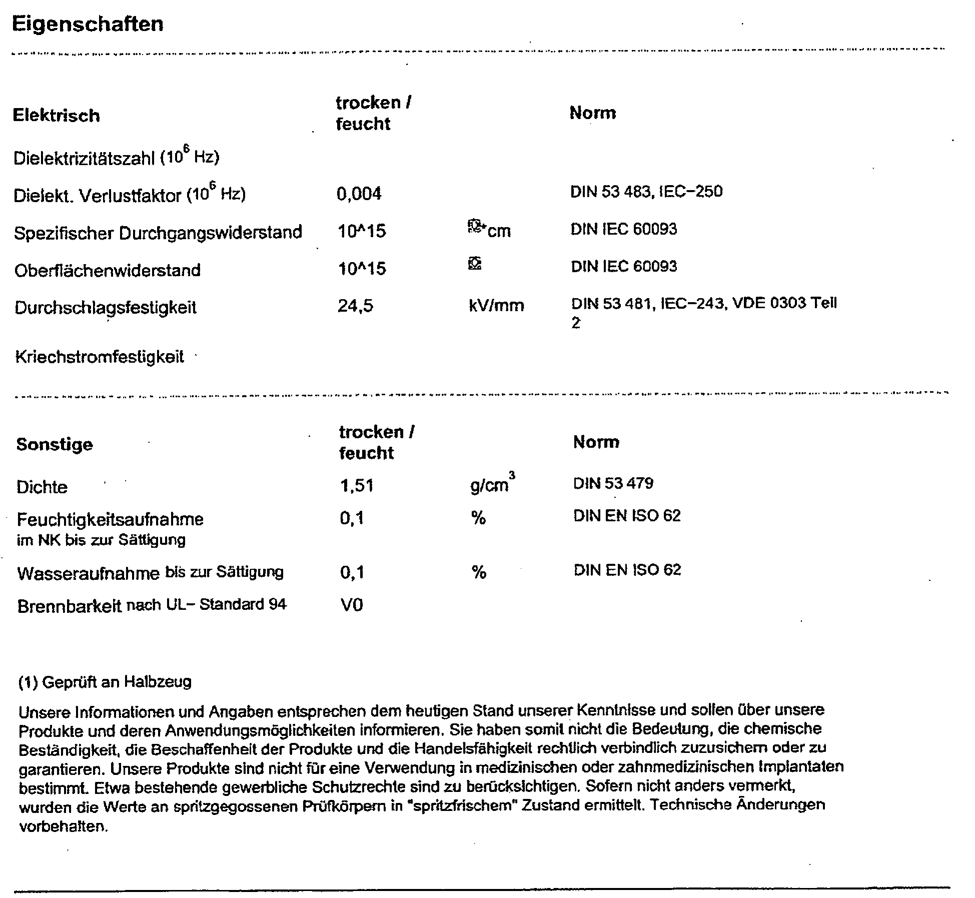

## Patentansprüche

1. Befestigungsmittel zur Lagefixierung eines Körpers, insbesondere eines Kopfes, für medizinische Zwecke, insbesondere bei Operationen, mit
• einem Halteabschnitt (8) und
• mit einem Eindringabschnitt, (9) der sich an den Halteabschnitt anschließt,
**dadurch gekennzeichnet, dass**
• der Eindringabschnitt einen ersten Bereich (9a) Form eines Kegelstumpfes, der an den Halteabschnitt angrenzt, und einen zweiten Bereich (9b) in Form eines Kegels aufweist, der die Spitze bildet, und
• sich der Winkel des Kegelstumpfmantels (α) bezüglich der Kegelstumpfachse von dem Winkel des Kegelmantels (β) bezüglich der Kegelachse unterscheidet.

2. Befestigungsmittel gemäß Anspruch 1, **dadurch gekennzeichnet, dass** das Befestigungsmittel wenigstens teilweise aus unsubstituiertem, einfach substituiertem oder mehrfach substituiertem Polyphenylen, insbesondere unsubstituiertem, einfach oder mehrfach substituiertem Polyparaphenylen, besteht,

3. Befestigungsmittel gemäß Anspruch 1, wobei das Befestigungsmittel wenigstens teilweise aus einem Polyparaphenylen-Copolymer besteht.

4. Befestigungsmittel gemäß einem der Ansprüche 1 bis 3, wobei das Befestigungsmittel wenigstens teilweise aus einem Polyparaphenylen-Copolymer besteht,
• wobei das erste Monomer einfach in ortho-Stellung substituiert ist,
• wobei das zweite Monomer einfach in meta-Stellung substituiert ist, und
• wobei der Substituent jeweils eine Benzoyl-Gruppe 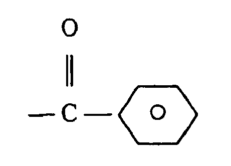
ist.

5. Befestigungsmittel gemäß Anspruch 1,
• **dadurch gekennzeichnet, dass** das Befestigungsmittel wenigstens teilweise aus einer Siliziumnitrid-Keramik, insbesondere aus einer β-Siliziumnitrid-Keramik, besteht.

6. Befestigungsmittel gemäß einem der Ansprüche 1 bis 5, bei dem der Eindringabschnitt und/oder der Halteabschnitt achsenrotationssymmetrisch ausgebildet ist.

7. Befestigungsmittel gemäß einem der Ansprüche 1 bis 6,
• wobei der Eindringabschnitt eine Spitze aufweist, und
• wobei der gesamte Eindringabschnitt nicht die Form eines Kegels hat.

8. Befestigungsmittel gemäß einem der Ansprüche 1 bis 7,
• wobei der Eindringabschnitt zweistückig ausgebildet ist, und
• wobei das eine Stück mit dem anderen Stück in festem Kontakt ist und wobei das eine Stück eine Spitze aufweist.

9. Befestigungsmittel gemäß den Ansprüchen 1 und 8, wobei die Spitze aus Keramik, insbesondere aus einer Siliziumnitrid-Keramik, besteht.

## Claims

1. A fastening means for fixing the position of a body, in particular a head, for medical purposes, in particular during operations, comprising:
• a holding portion (8); and
• a penetrating portion (9) connected to the holding portion;
**characterised in that**
• the penetrating portion comprises a first region (9a) in the form of a truncated cone which borders the holding portion, and a second region (9b) in the form of a cone which forms the tip, and
• the angle (α) of the surface of the truncated cone with respect to the axis of the truncated cone differs from the angle (β) of the surface of the cone with respect to the axis of the cone.

2. The fastening means in accordance with claim 1, **characterised in that** the fastening means at least partly consists of unsubstituted, monosubstituted or multisubstituted polyphenylene, in particular unsubstituted, monosubstituted or multisubstituted polyparaphenylene.

3. The fastening means in accordance with claim 1, wherein the fastening means at least partly consists of a polyparaphenylene copolymer.

4. The fastening means in accordance with any one of claims 1 to 3, wherein the fastening means at least partly consists of a polyparaphenylene copolymer, wherein:
• the first monomer is monosubstituted in the *ortho* position;
• the second monomer is monosubstituted in the *meta* position; and
• the substituent in each case is a benzoyl group

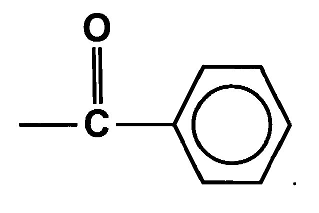

5. The fastening means in accordance with claim 1, **characterised in that** the fastening means at least partly consists of a silicon nitride ceramic, in particular a β silicon nitride ceramic.

6. The fastening means in accordance with any one of claims 1 to 5, wherein the penetrating portion and/or the holding portion is formed to be axially rotationally symmetrical.

7. The fastening means in accordance with any one of claims 1 to 6, wherein:
• the penetrating portion comprises a tip; and
• the penetrating portion as a whole does not have the shape of a cone.

8. The fastening means in accordance with any one of claims 1 to 7, wherein:
• the penetrating portion is formed in two pieces; and
• one piece is in firm contact with the other piece and comprises a tip.

9. The fastening means in accordance with claims 1 and 8, wherein the tip consists of ceramics, in particular a silicon nitride ceramic.

## Revendications

1. Moyen d'assujettissement, destiné à fixer en position un corps, en particulier une tête, à des fins médicales, en particulier lors d'opérations, comportant
- une partie maintien (8), et
- une partie pénétration (9), qui suit la partie maintien,
**caractérisé en ce que**
- la partie pénétration comprend un premier domaine (9a), ayant la forme d'un tronc de cône, qui est adjacent à la partie maintien, et un deuxième domaine (9b), ayant la forme d'un cône, qui forme la pointe, et
- l'angle α entre la surface latérale du tronc de cône et l'axe du tronc de cône est différent de l'angle β entre la surface latérale du cône et l'axe du cône.

2. Moyen d'assujettissement selon la revendication 1, **caractérisé en ce que** le moyen d'assujettissement est constitué au moins partiellement d'un polyphénylène non substitué, monosubstitué ou polysubstitué, en particulier d'un polyparaphénylène non substitué, monosubstitué ou polysubstitué.

3. Moyen d'assujettissement selon la revendication 1, le moyen d'assujettissement étant au moins partiellement constitué d'un copolymère de polyparaphénylène.

4. Moyen d'assujettissement selon l'une quelconque des revendications 1 à 3, le moyen d'assujettissement étant au moins partiellement constitué d'un copolymère de polyparaphénylène,
- le premier monomère étant monosubstitué en position ortho,
- le deuxième monomère étant monosubstitué en position méta, et
- le substituant étant dans chaque cas un groupe benzoyle

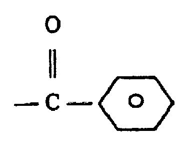

5. Moyen d'assujettissement selon la revendication 1, **caractérisé en ce qu'**il est au moins partiellement constitué d'une céramique de nitrure de silicium, en particulier d'une céramique de nitrure de silicium β.

6. Moyen d'assujettissement selon l'une quelconque des revendications 1 à 5, dans lequel la partie pénétration et/ou la partie maintien sont configurées en présentant une symétrie de rotation par rapport à l'axe.

7. Moyen d'assujettissement selon l'une quelconque des revendications 1 à 6,
- dans lequel la partie pénétration comporte une pointe, et
- dans lequel la partie pénétration, dans son ensemble, n'a pas la forme d'un cône.

8. Moyen d'assujettissement selon l'une quelconque des revendications 1 à 7,
- dans lequel la partie pénétration est réalisée en deux parties, et
- l'une des parties est en contact ferme avec l'autre partie, l'une des parties comportant une pointe.

9. Moyen d'assujettissement selon les revendications 1 à 8, dans lequel la pointe est en céramique, en particulier en une céramique de nitrure de silicium.
